(19) Europäisches Patentamt
European Patent Office
Office européen des brevets

(11) Veröffentlichungsnummer : **0 441 742 B1**

(12) # EUROPÄISCHE PATENTSCHRIFT

(45) Veröffentlichungstag der Patentschrift : **16.02.94 Patentblatt 94/07**

(51) Int. Cl.[5] : **C07C 323/65,** C07C 317/18, C08K 5/41, C09K 15/14, C10M 135/08

(21) Anmeldenummer : **91810060.3**

(22) Anmeldetag : **28.01.91**

(54) **Sulfoxide von bis- und tristhiomethylierten Phenolen.**

Die Akte enthält technische Angaben, die nach dem Eingang der Anmeldung eingereicht wurden und die nicht in dieser Patentschrift enthalten sind.

(30) Priorität : **05.02.90 CH 361/90**

(43) Veröffentlichungstag der Anmeldung : **14.08.91 Patentblatt 91/33**

(45) Bekanntmachung des Hinweises auf die Patenterteilung : **16.02.94 Patentblatt 94/07**

(84) Benannte Vertragsstaaten : **BE DE FR GB IT NL**

(56) Entgegenhaltungen :
**CH-A- 571 466**
**DE-A- 2 902 298**
**US-A- 4 874 885**
**Die Angewandte Makromolecular Chemie 82 (1979) 197-205**

(73) Patentinhaber : **CIBA-GEIGY AG**
**Klybeckstrasse 141**
**CH-4002 Basel (CH)**

(72) Erfinder : **Meier, Hans-Rudolf, Dr.**
**Rte du Confin 54**
**CH-1723 Marly (CH)**
Erfinder : **Pitteloud, Rita, Dr.**
**Sur le Village**
**CH-1724 Praroman (CH)**

EP 0 441 742 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

## Beschreibung

Die vorliegende Erfindung betrifft Sulfoxide von bis- und tris-thiomethylierten Phenolen, Mischungen dieser Sulfoxide mit den nicht oxidierten Verbindungen, diese Verbindungen oder Mischungen enthaltende Zusammensetzungen von organischen Polymeren, sowie die Verwendung der oben genannten Verbindungen und Mischungen als Stabilisatoren in Materialien, die gegen oxidativen, thermischen und/oder lichtinduzierten Abbau empfindlich sind.

Thiomethylierte Phenole werden als Stabilisatoren in organischen Polymeren eingesetzt. So beschreiben die US-A-3,660,352 und US-A-4,820,756 die Verwendung von 2,4,6-tri-substituierten Bis-(3,5-alkylthiomethyl)-phenolen als Antioxidantien in Elastomeren. Die US-A-3,227,677 offenbart 2,6-Bis-(alkoxycarbonylalkylthiomethyl)-4-alkylphenole als Antioxidantien für Polyolefine. Aus den US-A-4,857,572 und US-A-4,759,862 sind o,p-Alkylthiomethylphenole bekannt.

Weitere Alkylthiomethylphenole sind im US-A-4,874,885 und EP-A-273,013 veröffentlicht. Im US-A-4,741,846 werden 2,4,6-Tris-(alkylthiomethyl)-phenole beschrieben. In der Veröffentlichung von Jiráčková und Pospíšil in der Angewandten Makromolekularen Chemie 82 (1979), 197-205 werden Verbindungen wie 4,4'-Thio-bis(2-methyl-tert-butyl-phenol) und die entsprechenden am Schwefel mono- oder di-oxidierten Verbindungen als Antioxidantien untersucht.

Die JP-A-68-09,052, JP-A-68-16,741, JP-A-68-03,773 und JP-A-74-27,092 offenbaren Oxidationsprodukte von o-tert-butyl-substituierten alkylthiomethylierten Phenolen. In der GB-A-917,370 werden Bis-(dihydrocarbylhydroxybenzyl)-sulfide beschrieben.

Im Bereich der Antioxidantien für organische Polymere besteht weiterhin ein Bedarf an brauchbaren Verbindungen. Insbesondere im Bereich der Elastomere und Schmierstoffe erfordern neue Anwendungstechniken und neue Zusammensetzungen Anpassungen im Hinblick auf die Stabilisierung dieser Substrate. Es wurde nun gefunden, dass bestimmte Sulfoxide von bis- und tris-thiomethylierten Phenolen sehr gut zur Stabilisierung von gegen oxidativen, thermischen und/oder lichtinduzierten Abbau empfindlichen Materialien geeignet sind.

Gegenstand der vorliegenden Erfindung sind Verbindungen der Formeln I und II

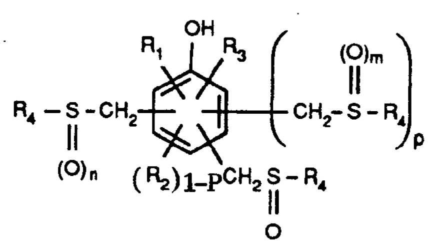

(I)

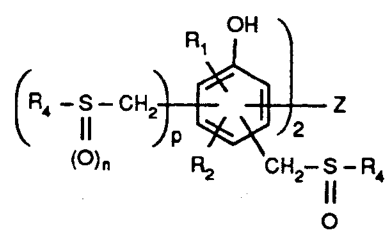

(II)

worin

$R_1$ Wasserstoff, $C_1$-$C_{18}$-Alkyl, Cyclohexyl oder $C_7$-$C_9$-Phenylalkyl bedeutet,

$R_2$ und $R_3$ unabhängig voneinander für Wasserstoff oder Methyl stehen,

$R_4$ für $C_4$-$C_{18}$-Alkyl, Phenyl, $C_7$-$C_9$-Phenylalkyl, mit $C_1$-$C_4$-Alkyl substituiertes Phenyl, Hydroxyethyl oder einen Rest $-(CH_2)_rCOOR_5$ steht, worin r 1 oder 2 entspricht und $R_5$ $C_1$-$C_{18}$-Alkyl bedeutet,

Z für Schwefel,

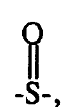

$>CR_2R_3$, wobei $R_2$ und $R_3$ die oben angegebene Bedeutung haben, oder für eine direkte Bindung steht und n, m und p unabhängig voneinander die Werte 0 oder 1 darstellen.

$R_1$ und $R_5$ als $C_1$-$C_{18}$-Alkyl können linear oder verzweigt sein und bedeuten beispielsweise Methyl, Ethyl, n-Propyl, Isopropyl, n-Butyl, tert-Butyl, n-Pentyl, Isopentyl, n-Hexyl, 2-Ethylhexyl, n-Heptyl, n-Octyl, i-Octyl, Decyl, Dodecyl, Tetradecyl oder Octadecyl.

Bevorzugt ist $R_1$ Wasserstoff oder $C_1$-$C_{12}$-Alkyl, z.B. $C_1$-$C_4$-Alkyl und als solches insbesondere Methyl, Ethyl, Isopropyl oder tert-Butyl und ganz besonders bevorzugt Methyl oder tert-Butyl.

$R_5$ ist bevorzugt $C_6$-$C_{12}$-Alkyl, z.B. $C_8$-$C_{12}$-Alkyl, insbesondere Octyl, z.B. 2-Ethylhexyl.

$R_1$ als $C_7$-$C_9$-Phenylalkyl steht z.B. für Benzyl, Phenylethyl, $\alpha$-Methylbenzyl, 3-Phenylpropyl, 2-Methylphenylethyl, 1-Methyl-phenylethyl oder $\alpha,\alpha$-Dimethylbenzyl. Bevorzugt ist Benzyl.

$R_4$ als $C_4$-$C_{18}$-Alkyl kann linear oder verzweigt sein und ist beispielsweise n-Butyl, tert-Butyl, n-Pentyl, Isopentyl, n-Hexyl, n-Heptyl, n-Octyl, i-Octyl, 2-Ethylhexyl, 1,1,3,3-Tetramethylbutyl, n-Nonyl, n-Decyl, n-Dodecyl, 1,1,3,3,5,5-Hexamethylhexyl oder 1,1,4,6,6-Pentamethylhept-(4)-yl.

Bevorzugt ist $R_4$ $C_6$-$C_{18}$-Alkyl, insbesondere $C_8$-$C_{12}$-Alkyl, z.B. n-Octyl, 1,1,3,3-Tetramethylbutyl oder n-Dodecyl.

$R_4$ als $C_7$-$C_9$-Phenylalkyl kann die gleichen Bedeutungsmöglichkeiten haben wie bei $R_1$ aufgezählt.

$R_4$ als mit $C_1$-$C_4$-Alkyl substituiertes Phenyl kann beispielsweise 1-3, insbesondere 1 oder 2 Alkylgruppen, vor allem Methylgruppen, enthalten. Beispiele dafür sind Tolyl, Xylyl und Mesityl. Bevorzugt ist $R_4$ $C_4$-$C_{18}$-Alkyl, Phenyl, $C_7$-$C_9$-Phenylalkyl oder $-CH_2COOR_5$ mit $R_5 = C_6$-$C_{12}$-Alkyl.

Z bedeutet insbesondere Methylen oder Schwefel. Der Substituent

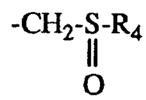

steht in Formel II vorzugsweise in m- oder p-Stellung zu Z, wobei p vorzugsweise 0 ist.

Von besonderem Interesse sind Verbindungen der Formeln I und II, worin p 0 ist, $R_4$ für $C_4$-$C_{18}$-Alkyl, Phenyl, $C_7$-$C_9$-Phenylalkyl, Hydroxyethyl oder einen Rest $-CH_2COOR_5$ steht, wobei $R_5$ $C_1$-$C_{18}$-Alkyl ist und $R_1$, $R_2$, $R_3$, Z, n und m die oben genannten Bedeutungen haben.

Bevorzugt sind Verbindungen der Formel I, insbesondere solche, worin p 0 ist, $R_1$ für Wasserstoff, $C_1$-$C_4$-Alkyl oder Cyclohexyl steht, $R_4$ $C_4$-$C_{18}$-Alkyl, insbesondere $C_8$-$C_{12}$-Alkyl, Benzyl, Phenyl, Hydroxyethyl oder $-CH_2COOR_5$ bedeutet, $R_5$ $C_1$-$C_{18}$-Alkyl entspricht und $R_2$, $R_3$, Z, n und m die zuvor genannten Bedeutungen haben.

Von besonderer Bedeutung sind Verbindungen der Formel I, worin p für 0 steht, die Gruppen

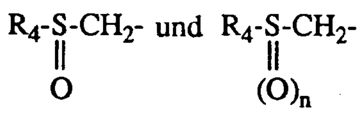

in 2,4- oder 2,6- oder 3,5-Stellung zur OH-Gruppe stehen und $R_1$, $R_2$, $R_3$, $R_4$, $R_5$, Z, n und m für die oben angegebenen Bedeutungsmöglichkeiten stehen.

Besonders bevorzugt sind Verbindungen der Formel I, worin $R_1$ Wasserstoff oder $C_1$-$C_4$-Alkyl ist, $R_4$ für ein $C_8$-$C_{12}$-Alkyl, Hydroxyethyl oder einen Rest $-CH_2COOR_5$ steht, wobei $R_5$ $C_8$-$C_{12}$-Alkyl bedeutet und $R_2$, $R_3$, Z, m, n und p die zuvor beschriebenen Bedeutungsmöglichkeiten haben und die Gruppen

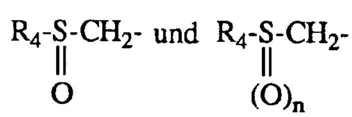

in 2,4- oder 2,6- oder 3,5-Stellung zur OH-Gruppe stehen.

Von ganz besonderem Interesse sind Verbindungen der Formel III

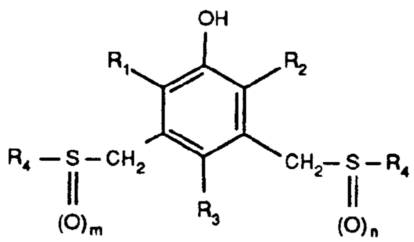

(III),

worin $R_1$ Wasserstoff, $C_1$-$C_4$-Alkyl oder Cyclohexyl ist, $R_2$, $R_3$, n und m für die unter Formel I angegebenen Bedeutungsmöglichkeiten stehen, wobei n + m ≧ 1 ist und $R_4$ für $C_4$-$C_{18}$-Alkyl, Phenyl, $C_7$-$C_9$-Phenylalkyl, Hydroxyethyl oder einen Rest $-CH_2COOR_5$ steht, wobei $R_5$ $C_1$-$C_{18}$-Alkyl ist.

Ebenfalls besonders bevorzugt sind Verbindungen der Formel IV

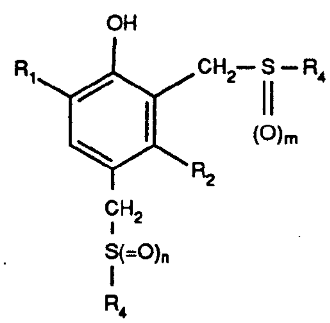

(IV),

worin $R_1$ $C_1$-$C_4$-Alkyl bedeutet, $R_2$, n und m für die unter Formel I angegebenen Bedeutungsmöglichkeiten stehen, wobei n + m ≧ 1 ist und $R_4$ die gleiche Bedeutung wie in Formel III hat.

Ausserdem sind Verbindungen der Formel V besonders bevorzugt

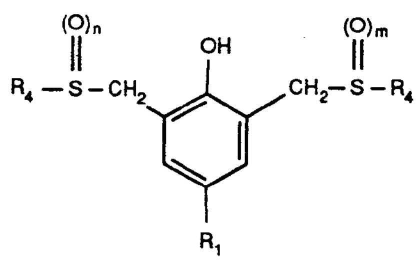

(V),

worin $R_1$ für $C_1$-$C_{12}$-Alkyl steht, n und m wie unter Formel I beschrieben definiert sind, wobei n + m ≧ 1 ist und $R_4$ die gleiche Bedeutung wie in Formel III hat.

Ein weiterer Gegenstand der Erfindung sind Mischungen von mindestens einer Verbindung der Formel I und/oder II mit mindestens einer Verbindung der Formel VI und/oder VII

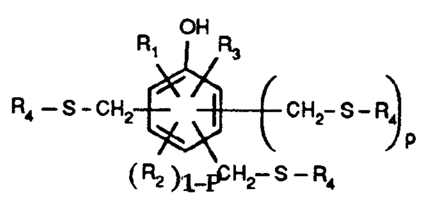

(VI)

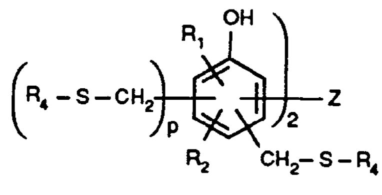

(VII),

worin $R_1$, $R_2$, $R_3$, $R_4$, Z und p die oben für die Formeln I und II angegebenen Bedeutungen haben. Bevorzugte Bedeutungen entsprechen jenen, die für gleichnamige Substituenten ebenfalls weiter oben angeführt sind.

Bevorzugt sind Mischungen, worin sich die Komponenten der Formeln I und II von jenen der Formeln VI und VII nur dadurch unterscheiden, dass letztere an Stelle der

$-\overset{O}{\overset{\|}{S}}$-Gruppierungen

-S-Gruppen enthalten, sonst aber die gleiche Struktur besitzen wie erstere.

Weiterhin bevorzugt sind Mischungen, die durch partielle Oxidation von mindestens einer Verbindung der Formel VI und/oder VII erhalten werden.

Das Verhältnis der Mischungen von mindestens einer Verbindung der Formel I und/oder II mit mindestens einer Verbindung der Formel VI und/oder VII beträgt 0.5:99.5 bis 99.5:0.5. Bevorzugt sind Mischungsverhältnisse von 90:10 bis 10:90, insbesondere von 80:20 bis 20:80.

Die erfindungsgemässen Verbindungen der Formeln I und II bzw. die Mischungen aus diesen Verbindungen und Verbindungen der Formeln VI und VII eignen sich hervorragend zum Stabilisieren von gegen oxidativen, thermischen und/oder lichtinduzierten Abbau empfindlichen organischen Materialien.

Ein weiterer Gegenstand der Erfindung sind daher Zusammensetzungen, die ein gegen oxidativen, thermischen und/oder lichtinduzierten Abbau empfindliches organisches Material und mindestens eine Verbindung der Formel I und/oder II oder eine Mischung von mindestens einer Verbindung der Formel I und/oder II mit mindestens einer Verbindung der Formel VI und/oder VII enthalten, sowie die Verwendung der genannten Verbindungen und Mischungen zum Stabilisieren von gegen oxidativen, thermischen und/oder lichtinduzierten Abbau empfindlichen Materialien. Als Beispiele von derartigen organischen Materialien, die in den erfindungsgemässen Zusammensetzungen enthalten sind und die erfindungsgemäss stabilisiert werden können, seien beispielsweise die folgenden erwähnt:

1. Polymere von Mono- und Diolefinen, beispielsweise Polypropylen, Polyisobutylen, Polybuten-1, Polymethylpenten-1, Polyisopren oder Polybutadien sowie Polymerisate von Cycloolefinen wie z.B. von Cyclopenten oder Norbornen; ferner Polyethylen (das gegebenenfalls vernetzt sein kann), z.B. Polyethylen hoher Dichte (HDPE), Polyethylen niederer Dichte (LDPE), lineares Polyethylen niederer Dichte (LLDPE).

2. Mischungen der unter 1) genannten Polymeren, z.B. Mischungen von Polypropylen mit Polyisobutylen, Polypropylen mit Polyethylen (z.B. PP/HDPE, PP/LDPE) und Mischungen verschiedener Polyethylentypen (z.B. LDPE/HDPE).

3. Copolymere von Mono- und Diolefinen untereinander oder mit anderen Vinylmonomeren, wie z.B. Ethylen-Propylen-Copolymere, lineares Polyethylen niederer Dichte (LLDPE) und Mischungen desselben mit Polyethylen niederer Dichte (LDPE), Propylen-Buten-1-Copolymere, Propylen-Isobutylen-Copolymere, Ethylen-Buten-1-Copolymere, Ethylen-Hexen-Copolymere, Ethylen-Methylpenten-Copolymere, Ethylen-Hepten-Copolymere, Ethylen-Octen-Copolymere, Propylen-Butadien-Copolymere, Isobutylen-Isopren-Copolymere, Ethylen-Alkylacrylat-Copolymere, Ethylen-Alkylmethacrylat-Copolymere, Ethylen-Vinylacetat-Copolymere oder Ethylen-Acrylsäure-Copolymere und deren Salze (Ionomere), sowie Terpolymere von Ethylen mit Propylen und einem Dien, wie Hexadien, Dicyclopentadien oder Ethylidennorbornen; ferner Mischungen solcher Copolymere untereinander und mit unter 1) genannten Polymeren, z.B. Polypropylen/Ethylen-Propylen-Copolymere, LDPE/Ethylen-Vinylacetat-Copolymere, LDPE/Ethylen-Acrylsäure-Copolymere, LLDPE/Ethylen-Vinylacetat-Copolymere und LLDPE/Ethylen-Acrylsäure-Copolymere.

3a. Kohlenwasserstoffharze (z.B. $C_5$-$C_9$) inklusive hydrierte Modifikationen davon (z.B. Klebrigmacherharze).

4. Polystyrol, Poly-(p-methylstyrol), Poly-($\alpha$-methylstyrol).

5. Copolymere von Styrol oder $\alpha$-Methylstyrol mit Dienen oder Acrylderivaten, wie z.B. Styrol-Butadien, Styrol-Acrylnitril, Styrol-Alkylimethacrylat, Styrol-Butadien-Alkylacrylat, Styrol-Maleinsäureanhydrid, Styrol-Acrylnitril-Methylacrylat; Mischungen von hoher Schlagzähigkeit aus Styrol-Copolymeren und einem anderen Polymer, wie z.B. einem Polyacrylat, einem Dien-Polymeren oder einem Ethylen-Propylen-Dien-Terpolymeren; sowie Block-Copolymere des Styrols, wie z.B. Styrol-Butadien-Styrol, Styrol-Isopren-Styrol, Styrol-Ethylen/Butylen-Styrol oder Styrol-Ethylen/Propylen-Styrol.

6. Pfropfcopolymere von Styrol oder a-Methylstyrol, wie z.B. Styrol auf Polybutadien, Styrol auf Polybutadien-Styrol- oder Polybutadien-Acrylnitril-Copolymere, Styrol und Acrylnitril (bzw. Methacrylnitril) auf Polybutadien; Styrol, Acrylnitril und Methylmethacrylat auf Polybutadien; Styrol und Maleinsäureanhydrid auf Polybutadien; Styrol, Acrylnitril und Maleinsäureanhydrid oder Maleinsäureimid auf Polybutadien; Styrol und Maleinsäureimid auf Polybutadien, Styrol und Alkylacrylate bzw. Alkylmethacrylate auf Polybutadien, Styrol und Acrylnitril auf Ethylen-Propylen-Dien-Terpolymeren, Styrol und Acrylnitril auf Polyalkylacrylaten oder Polyalkylmethacrylaten, Styrol und Acrylnitril auf Acrylat-Butadien-Copolymeren, sowie deren Mischungen mit den unter 5) genannten Copolymeren, wie sie z.B. als sogenannte ABS-, MBS-, ASA- oder AES-Polymere bekannt sind.

7. Halogenhaltige Polymere, wie z.B. Polychloropren, Chlorkautschuk, chloriertes oder chlorsulfoniertes Polyethylen, Copolymere von Ethylen und chloriertem Ethylen, Epichlorhydrinhomo- und -copolymere, insbesondere Polymere aus halogenhaltigen Vinylverbindungen, wie z.B. Polyvinylchlorid, Polyvinylidenchlorid, Polyvinylfluorid, Polyvinylidenfluorid; sowie deren Copolymere, wie Vinylchlorid-Vinylidenchlorid, Vinylchlorid-Vinylacetat oder Vinylidenchlorid-Vinylacetat.

8. Polymere, die sich von $\alpha,\beta$-ungesättigten Säuren und deren Derivaten ableiten, wie Polyacrylate und Polymethacrylate, Polyacrylamide und Polyacrylnitrile.

9. Copolymere der unter 8) genannten Monomeren untereinander oder mit anderen ungesättigten Monomeren, wie z.B. Acrylnitril-Butadien-Copolymere, Acrylnitril-Alkylacrylat-Copolymere, Acrylnitril-Alkoxyalkylacrylat-Copolymere, Acrylnitril-Vinylhalogenid-Copolymere oder Acrylnitril-Alkylmethacrylat-Butadien-Terpolymere.

10. Polymere, die sich von ungesättigten Alkoholen und Aminen bzw. deren Acylderivaten oder Acetalen ableiten, wie Polyvinylalkohol, Polyvinylacetat, -stearat, -benzoat, -maleat, Polyvinylbutyral, Polyallylphthalat, Polyallylmelamin; sowie deren Copolymere mit in Punkt 1 genannten Olefinen.

11. Homo- und Copolymere von cyclischen Ethern, wie Polyalkylenglykole, Polyethylenoxyd, Polypropylenoxyd oder deren Copolymere mit Bisglycidylethern.

12. Polyacetale, wie Polyoxymethylen, sowie solche Polyoxymethylene, die Comonomere, wie z.B. Ethylenoxid, enthalten; Polyacetale, die mit thermoplastischen Polyurethanen, Acrylaten oder MBS modifiziert sind.

13. Polyphenylenoxide und -sulfide und deren Mischungen mit Styrolpolymeren oder Polyamiden.

14. Polyurethane, die sich von Polyethern, Polyestern und Polybutadienen mit endständigen Hydroxylgruppen einerseits und aliphatischen oder aromatischen Polyisocyanaten andererseits ableiten, sowie deren Vorprodukte.

15. Polyamide und Copolyamide, die sich von Diaminen und Dicarbonsäuren und/oder von Aminocarbonsäuren oder den entsprechenden Lactamen ableiten, wie Polyamid 4, Polyamid 6, Polyamid 6/6, 6/10, 6/9, 6/12, 4/6, Polyamid 11, Polyamid 12, aromatische Polyamide ausgehend von m-Xylol, Diamin und Adipinsäure; Polyamide, hergestellt aus Hexamethylendiamin und Iso- und/oder Terephthalsäure und gegebenenfalls einem Elastomer als Modifikator, z.B. Poly-2,4,4-trimethylhexamethylenterephthalamid, Poly-m-phenylen-isophthalamid. Block-Copolymere der vorstehend genanntenPolyamide mit Polyolefinen, Olefin-Copolymeren, Ionomeren oder chemisch gebundenen oder gepfropften Elastomeren; oder mit Polyethern, wie z.B. mit Polyethylenglykol, Polypropylenglykol oder Polytetramethylenglykol. Ferner mit EPDM oder ABS modifizierte Polyamide oder Copolyamide; sowie während der Verarbeitung kondensierte Polyamide ("RIM-Polyamid-systeme").

16. Polyharnstoffe, Polyimide, Polyamid-imide und Polybenzimidazole.

17. Polyester, die sich von Dicarbonsäuren und Dialkoholen und/oder von Hydroxycarbonsäuren oder den entsprechenden Lactonen ableiten, wie Polyethylenterephthalat, Polybutylenterephthalat, Poly-1,4-dimethylolcyclohexanterephthalat, Polyhydroxybenzoate, sowie Block-Polyether-ester, die sich von Polyethern mit Hydroxylendgruppen ableiten; ferner mit Polycarbonaten oder MBS modifizierte Polyester.

18. Polycarbonate und Polyestercarbonate.

19. Polysulfone, Polyethersulfone und Polyetherketone.

20. Vernetzte Polymere, die sich von Aldehyden einerseits und Phenolen, Harnstoff oder Melamin andererseits ableiten, wie Phenol-Formaldehyd-, Harnstoff-Formaldehyd- und Melamin-Formaldehydharze.

21. Trocknende und nicht-trocknende Alkydharze.

22. Ungesättigte Polyesterharze, die sich von Copolyestern gesättigter und ungesättigter Dicarbonsäuren mit mehrwertigen Alkoholen, sowie Vinylverbindungen als Vernetzungsmittel ableiten, wie auch deren halogenhaltige, schwerbrennbare Modifikationen.

23. Vernetzbare Acrylharze, die sich von substituierten Acrylsäureestern ableiten, wie z.B. von Epoxyacrylaten, Urethan-acrylaten oder Polyester-acrylaten.

24. Alkydharze, Polyesterharze und Acrylatharze, die mit Melaminharzen, Harnstoffharzen, Polyisocyanaten oder Epoxidharzen vernetzt sind.

25. Vernetzte Epoxidharze, die sich von Polyepoxiden ableiten, z.B. von Bis-glycidylethern oder von cycloaliphatischen Diepoxiden.

26. Natürliche Polymere, wie Cellulose, Naturkautschuk, Gelatine, sowie deren polymerhomolog chemisch abgewandelte Derivate, wie Celluloseacetate, -propionate und -butyrate, bzw. die Celluloseether, wie Methylcellulose; sowie Kolophoniumharze und Derivate.

27. Mischungen (Polyblends) der vorgenannten Polymeren, wie z.B. PP/EPDM, Polyamid/EPDM oder ABS, PVC/EVA, PVC/ABS, PVC/MBS, PC/ABS, PBTP/ABS, PC/ASA, PC/PBT, PVC/CPE, PVC/Acrylate, POM/thermoplastisches PUR, PC/thermoplastisches PUR, POM/Acrylat, POM/MBS, PPO/HIPS, PPO/PA 6.6 und Copolymere, PA/HDPE, PA/PP, PA/PPO.

28. Natürliche und synthetische organische Stoffe, die reine monomere Verbindungen oder Mischungen von solchen darstellen, beispielsweise Mineralöle, tierische oder pflanzliche Fette, Oele und Wachse, oder Oele, Wachse und Fette auf Basis synthetischer Ester (z.B. Phthalate, Adipate, Phosphate oder Trimellitate), sowie Abmischungen synthetischer Ester mit Mineralölen in beliebigen Gewichtsverhältnissen, wie sie z.B. als Spinnpräparationen Anwendung finden, sowie deren wässrige Emulsionen.

29. Wässrige Emulsionen natürlicher oder synthetischer Kautschuke, wie z.B. Naturkautschuk-Latex oder Latices von carboxylierten Styrol-Butadien-Copolymeren.

Die Zusammensetzungen enthalten zweckmässig 0,01-10 Gew.-% der erfindungsgemässen Verbindungen bzw. Mischungen, bezogen auf das organische Material, insbesondere 0,05-5,0 Gew.-%, vorzugsweise 0,05-3 Gew.-%, z.B. 0,1-2 Gew.-%.

Bei dem gegen oxidativen, thermischen und/oder lichtinduzierten Abbau empfindlichen Material kann es sich also beispielsweise um ein organisches Polymer, einen Schmierstoff oder eine Hydraulikflüssigkeit handeln. Bei den organischen Polymeren handelt es sich dabei vorzugsweise um synthetische Polymere, insbesondere um Elastomere.

Besonders bevorzugte Zusammensetzungen enthalten Elastomere oder Schmierstoffe und mindestens eine Verbindung der Formel I oder Mischungen von mindestens einer Verbindung der Formel I mit mindestens einer Verbindung der Formel VI.

Die in Frage kommenden Schmierstoffe basieren beispielsweise auf mineralischen oder synthetischen Oelen oder Mischungen davon. Die Schmierstoffe sind dem Fachmann geläufig und in der einschlägigen Fachliteratur, wie beispielsweise in Dieter Klamann, "Schmierstoffe und verwandte Produkte" (Verlag Chemie, Weinheim, 1982), in Schewe-Kobek, "Das Schmiermittel-Taschenbuch" (Dr. Alfred Hüthig-Verlag, Heidelberg, 1974) und in "Ullmanns Enzyklopädie der technischen Chemie", Bd. 13, Seiten 85-94 (Verlag Chemie, Weinheim, 1977) beschrieben.

Die Schmierstoffe sind insbesondere Oele und Fette, beispielsweise basierend auf einem Mineralöl. Bevorzugt sind Oele.

Eine weitere Gruppe von Schmierstoffen, die zur Anwendung gelangen können, sind pflanzliche oder tierische Oele, Fette, Talge und Wachse oder deren Gemische untereinander oder Gemische mit den erwähnten mineralischen oder synthetischen Oelen. Pflanzliche und tierische Oele, Fette, Talge und Wachse sind beispielsweise Palmkernöl, Palmöl, Olivenöl, Rueböl, Rapsöl, Leinöl, Erdnussöl, Sojabohnenöl, Baumwollöl, Sonnenblumenöl, Kürbiskernöl, Kokosnussöl, Maisöl, Rizinusöl, Baumnussöl und Mischungen davon, Fischöle, Talge von Schlachttieren wie Rindertalg, Klauenfett und Knochenöl sowie deren modifizierte, epoxidierte und sulfoxidierte Formen, beispielsweise epoxidiertes Sojabohnenöl.

Die Mineralöle basieren insbesondere auf Kohlenwasserstoffverbindungen.

Beispiele von synthetischen Schmierstoffen umfassen Schmierstoffe auf der Basis der aliphatischen oder aromatischen Carboxylester, der polymeren Ester, der Polyalkylenoxide, der Phosphorsäureester, der Poly-$\alpha$-olefine oder der Silicone, eines Diesters einer zweiwertigen Säure mit einem einwertigen Alkohol, wie z.B. Dioctylsebacat oder Dinonyladipat, eines Triesters von Trimethylolpropan mit einer einwertigen Säure oder mit einem Gemisch solcher Säuren, wie z.B. Trimethylolpropantripelargonat, Trimethylolpropan-tricaprylat oder Gemische davon, eines Tetraesters von Pentaerythrit mit einer einwertigen Säure oder mit einem Gemisch solcher Säuren, wie z.B. Pentaerythrit-tetracaprylat, oder eines komplexen Esters von einwertigen und zweiwertigen Säuren mit mehrwertigen Alkoholen, z.B. ein komplexer Ester von Trimethylolpropan mit Capryl- und Sebacinsäure oder von einem Gemisch davon. Besonders geeignet sind neben Mineralölen z.B. Poly-$\alpha$-Olefine, Schmierstoffe auf Esterbasis, Phosphate, Glykole, Polyglykole und Polyalkylenglykole, sowie deren Mischungen mit Wasser.

Als Elastomere können die erfindungsgemässen Zusammensetzungen beispielsweise folgende Materialien enthalten:

1. Polydiene, wie beispielsweise Polybutadien, Polyisopren oder Polychloropren; Blockpolymere, wie beispielsweise Styrol/Butadien/Styrol, Styrol/Isopren/Styrol oder Acrylnitril/Butadien-Copolymere.

2. Copolymere von Mono- und Diolefinen untereinander oder mit anderen Vinylmonomeren, wie z.B. Aethylen-Alkylacrylat-Copolymere, Aethylen-Alkylmethacrylat-Copolymere, Aethylen-Vinylacetat-Copolymere sowie Terpolymere von Aethylen mit Propylen und einem Dien, wie Hexadien, Dicyclopentadien oder Aethylidennorbornen.

3. Halogenhaltige Polymere, wie z.B. Polychloropren, Chlorkautschuk, chloriertes oder chlorsulfoniertes Polyäthylen, Epichlorhydrin-Homo- und -Copolymere, Chlortrifluoroäthylen-Copolymere, Polymere aus halogenhaltigen Vinylverbindungen, wie z.B. Polyvinylidenchlorid, Polyvinylidenfluorid; sowie deren Copolymere, wie Vinylchlorid-Vinylidenchlorid, Vinylchlorid-Vinylacetat oder Vinylidenchlorid-Vinylacetat.

4. Polyurethane, die sich von Polyäthern, Polyestern und Polybutadien mit endständigen Hydroxylgruppen einerseits und aliphatischen oder aromatischen Polyisocyanaten andererseits ableiten sowie deren Vorprodukte.

5. Naturkautschuk.

6. Mischungen (Polyblends) der vorgenannten Polymeren.

7. Wässrige Emulsionen natürlicher oder synthetischer Kautschuke, wie z.B. Naturkautschuk-Latex oder Latices von carboxylierten Styrol-Butadien-Copolymeren.

Gegebenenfalls liegen diese Elastomere als Latices vor und können als solche stabilisiert werden.

Bevorzugt sind Zusammensetzungen, welche als Elastomeres ein Polydien, wie Polybutadien-Kautschuk, ein halogenhaltiges Polymer, wie Polyvinylidenfluorid, oder ein Polyurethan enthalten.

Die Einarbeitung in die organischen Materialien kann beispielsweise durch Einmischen der erfindungsgemässen Verbindungen oder Mischungen und gegebenenfalls weiterer Additive nach den in der Technik üblichen Methoden erfolgen. Handelt es sich um Polymere, insbesondere synthetische Polymere, kann die Einarbeitung vor oder während der Formgebung, oder durch Aufbringen der gelösten oder dispergierten Verbindungen auf das Polymere, gegebenenfalls unter nachträglichem Verdunsten des Lösungsmittels erfolgen. Im Fall von Elastomeren können diese auch als Latices stabilisiert werden. Eine weitere Möglichkeit der Einarbeitung der erfindungsgemässen Verbindungen bzw. Mischungen in Polymere besteht in deren Zugabe vor oder während der Polymerisation der entsprechenden Monomeren bzw. vor der Vernetzung. Im Falle der Zugabe vor oder während der Polymerisation können die Verbindungen der Formel I oder II bzw. die erfindungsgemässen Mischungen aus Verbindungen der Formel I und/oder II mit mindestens einer Verbindung der Formel VI und/oder VII auch als Regulatoren für die Kettenlänge der Polymerisation (Kettenabbrecher) wirken.

Die erfindungsgemässen Verbindungen oder Mischungen können auch in Form eines Masterbatches, der diese Verbindungen beispielsweise in einer Konzentration von 2,5 bis 25 Gew.-% enthält, den zu stabilisierenden Kunststoffen zugesetzt werden.

Erfindungsgemässe Polymerzusammensetzungen können in verschiedener Form angewendet bzw. zu verschiedenen Produkten verarbeitet werden, z.B. als (zu) Folien, Fasern, Bändchen, Formmassen, Profilen, oder als Bindemittel für Lacke, Klebstoffe oder Kitte.

Erfindungsgemässe Schmierstoffzusammensetzungen finden z.B. Verwendung in Verbrennungsmotoren, z.B. in Kraftfahrzeugen.

Zusätzlich zu den erfindungsgemässen Verbindungen bzw. Mischungen können die erfindungsgemässen Zusammensetzungen, insbesondere wenn sie organische, vorzugsweise synthetische, Polymere enthalten, noch weitere übliche Additive enthalten. Beispiele für solche Additive sind:

1. Antioxidantien

1.1. Alkylierte Monophenole, z.B. 2,6-Di-tert.butyl-4-methylphenol, 2-Tert.butyl-4,6-dimethylphenol, 2,6-Di-tert.butyl-4-ethylphenol, 2,6-Di-tert.butyl-4-n-butylphenol, 2,6-Di-tert.butyl-4-i-butylphenol, 2,6-Di-cyclopentyl-4-methylphenol, 2-(α-Methylcyclohexyl)-4,6-dimethylphenol, 2,6-Di-octadecyl-4-methylphenol, 2,4,6-Tri-cyclohexylphenol, 2,6-Di-tert.butyl-4-methoxymethylphenol, 2,6-Di-nonyl-4-methylphenol.

1.2. Alkylierte Hydrochinone, z.B. 2,6-Di-tert.butyl-4-methoxyphenol, 2,5-Di-tert.butyl-hydrochinon, 2,5-Di-tert.amyl-hydrochinon, 2,6-Diphenyl-4-octadecyloxyphenol.

1.3. Hydroxylierte Thiodiphenylether, z.B. 2,2'-Thio-bis-(6-tert.-butyl-4-methylphenol), 2,2'-Thio-bis-(4-octylphenol), 4,4'-Thio-bis-(6-tert.butyl-3-methylphenol), 4,4'-Thio-bis-(6-tert.butyl-2-methylphenol).

1.4. Alkyliden-Bisphenole, z.B. 2,2'-Methylen-bis-(6-tert.butyl-4-methylphenol), 2,2'-Methylen-bis-(6-tert.butyl-4-ethylphenol), 2,2'-Methylen-bis-[4-methyl-6-(α-methylcyclohexyl)-phenol], 2,2'-Methylen-bis-(4-methyl-6-cyclohexylphenol), 2,2'-Methylen-bis-(6-nonyl-4-methylphenol), 2,2'-Methylen-bis-(4,6-di-tert.butylphenol), 2,2'-Ethyliden-bis-(4,6-di-tert.butylphenol), 2,2'-Ethyliden-bis-(6-tert.butyl-4-isobutylphenol), 2,2'-Methylen-bis-[6-(α-methylbenzyl)-4-nonylphenol], 2,2'-Methylen-bis-[6-(α,α-dimethylbenzyl)-4-nonylphenol], 4,4'-Methylen-bis-(2,6-di-tert.-butylphenol), 4,4'-Methylen-bis-(6-tert.butyl-2-methylphenol), 1,1-Bis-(5-tert.butyl-4-hydroxy-2-methyl-phenyl)-butan, 2,6-Bis-(3-tert.butyl-5-methyl-2-hydroxybenzyl)-4-methylphenol, 1,1,3-Tris-(5-tert.butyl-4-hydroxy-2-methylphenyl)-butan, 1,1-Bis-(5-tert.butyl-4-hydroxy-2-methyl-phenyl)-3-n-dodecylmercaptobutan, Ethylenglycol-bis-[3,3-bis-(3'-tert.-butyl-4'-hydroxyphenyl)-butyrat], Bis-(3-tert.butyl-4-hydroxy-5-methyl-phenyl)-dicyclopentadien, Bis-[2-(3'-tert.butyl-2'-hydroxy-5'-methyl-benzyl)-6-tert.butyl-4-methyl-phenyl]-terephthalat.

1.5 Benzylverbindungen, z.B. 1,3,5-Tris-(3,5-di-tert.butyl-4-hydroxy-benzyl)-2,4,6-tri-methylbenzol, Bis-(3,5-di-tert.butyl-4-hydroxybenzyl)-sulfid, 3,5-Di-tert.butyl-4-hydroxybenzyl-mercaptoessigsäure-isooctylester, Bis-(4-tert.butyl-3-hydroxy-2,6-dimethylbenzyl)dithiol-terephthalat, 1,3,5-Tris-(3,5-di-tert.butyl-4-hydroxybenzyl)-isocyanurat, 1,3,5-Tris-(4-tert.butyl-3-hydroxy-2,6-dimethylbenzyl)-isocyanurat, 3,5-Di-tert.butyl-4-hydroxybenzyl-phosphonsäure-dioctadecylester, Ca-Salz des 3,5-Di-tert.butyl-4-hydroxybenzyl-phosphonsäure-monoethylester, 1,3,5-Tris-(3,5-dicyclohexyl-4-hydroxybenzyl)isocyanurat.

1.6. Acylaminophenole, z.B. 4-Hydroxy-laurinsäureanilid, 4-Hydroxystearinsäureanilid, 2,4-Bis-(octylmercapto)-6-(3,5-di-tert.butyl-4-hydroxyanilino )-s-triazin, N-(3,5-di-tert.-butyl-4-hydroxyphenyl)-carbaminsäureoctylester.

1.7. Ester der β-(3,5-Di-tert.butyl-4-hydroxyphenyl)-propionsäure mit ein- oder mehrwertigen Alkoholen, wie z.B. mit Methanol, Octa- decanol, 1,6-Hexandiol, Neopentylglycol, Thiodiethylenglycol, Diethylenglycol, Triethylenglycol, Pentaerythrit, Tris-(hydroxyethyl)-isocyanurat, N,N'-Bis-(hydroxyethyl)-oxalsäurediamid.

1.8. Ester der β-(5-tert.Butyl-4-hydroxy-3-methylphenyl)-propionsäure mit ein- oder mehrwertigen Alkoholen, wie z.B. mit Methanol, Octadecanol, 1,6-Hexandiol, Neopentylglycol, Thiodiethylenglycol, Diethylenglycol, Triethylenglycol, Pentaerythrit, Tris-(hydroxy)ethyl-isocyanurat, N,N'-Bis-(hydroxyethyl)-oxalsäurediamid.

1.9. Ester der β-(3,5-Dicyclohexyl-4-hydroxyphenyl)-propionsäure mit ein- oder mehrwertigen Alkoholen, wie z.B. mit Methanol, Octadecanol, 1,6-Hexandiol, Neopentylglycol, Thiodiethylenglycol, Diethylenglycol, Triethylenglycol, Pentaerythrit, Tris-(hydroxy)ethyl-isocyanurat, N,N'-Bis-(hydroxyethyl)-oxalsäurediamid.

1.10. Amide der β-(3,5-Di-tert.butyl-4-hydroxyphenyl)-propionsäure, wie z.B. N,N'-Bis-(3,5-di-tert.butyl-4-hydroxyphenylpropionyl)-hexamethylendiamin, N,N'-Bis-(3,5-di-tert.butyl-4-hydroxyphenylpropionyl)-trimethylendiamin, N,N'-Bis-(3,5-di-tert.butyl-4-hydroxyphenylpropionyl)-hydrazin.

2. UV-Absorber und Lichtschutzmittel

2.1. 2-(2'-Hydroxyphenyl)-benztriazole, wie z.B. das 5'-Methyl-, 3',5'-Di-tert.butyl-, 5'-tert.Butyl-, 5'-(1,1,3,3-Tetramethylbutyl)-, 5-Chlor-3',5'-di-tert.butyl-, 5-Chlor-3'-tert.butyl-5'-methyl-, 3'-sec.Butyl-5'-tert.butyl, 4'-Octoxy-, 3',5'-Di-tert.amyl-, 3',5'-Bis-(α,α-dimethylbenzyl)-Derivat.

2.2. 2-Hydroxybenzophenone, wie z.B. das 4-Hydroxy-, 4-Methoxy-, 4-Octoxy-, 4-Decyloxy-, 4-Dodecyloxy-, 4-Benzyloxy-, 4,2',4'-Trihydroxy-, 2'-Hydroxy-4,4'-dimethoxy-Derivat.

2.3. Ester von gegebenenfalls substituierten Benzoesäuren, wie z.B. 4-tert.Butyl-phenylsalicylat, Phenylsalicylat, Octylphenyl-salicylat, Dibenzoylresorcin, Bis-(4-tert.butylbenzoyl)-resorcin, Benzoylresorcin, 3,5-Di-tert.butyl-4-hydroxybenzoesäure-2,4-di-tert.-butylphenylester, 3,5-Di-tert.butyl-4-hydroxybenzoesäurehexadecylester.

2.4. Acrylate, wie z.B.α-Cyan-β,β-diphenylacrylsäure-ethylester bzw. -isooctylester, α-Carbomethoxy-zimtsäuremethylester, α-Cyano-β-methyl-p-methoxy-zimtsäuremethylester bzw. -butylester, α-Carbomethoxy-p-methoxy-zimtsäure-methylester, N-(β-Carbomethoxy-β-cyanovinyl)-2-methyl-indolin.

2.5. Nickelverbindungen, wie z.B. Nickelkomplexe des 2,2'-Thio-bis-[4-(1,1,3,3-tetramethylbutyl)-phenols], wie der 1:1- oder der 1:2-Komplex, gegebenenfalls mit zusätzlichen Liganden, wie n-Butylamin, Triethanolamin oder N-Cyclohexyl-diethanolamin, Nickeldibutyldithiocarbamat, Nickelsalze von 4-Hydroxy-3,5-di-tert.butylbenzyl-phosphonsäure-monoalkylestern, wie vom Methyl- oder Ethylester, Nickelkomplexe von Ketoximen, wie von 2-Hydroxy-4-methyl-phenyl-undecylketoxim, Nickelkomplexe des 1-Phenyl-4-lauroyl-5-hydroxy-pyrazols, gegebenenfalls mit zusätzlichen Liganden.

2.6. Sterisch gehinderte Amine, wie z.B. Bis-(2,2,6,6-tetramethyl-piperidyl)-sebacat, Bis-(1,2,2,6,6-pentamethylpiperidyl)-sebacat, n-Butyl-3,5-di-tert.butyl-4-hydroxybenzyl-malonsäure-bis(1,2,2,6,6-pentamethylpiperidyl)-ester, Kondensationsprodukt aus 1-Hydroxyethyl-2,2,6,6-tetramethyl-4-hydroxypiperidin und Bernsteinsäure, Kondensationsprodukt aus N,N'-Bis-(2,2,6,6-Tetramethyl-4-piperidyl)-hexamethylendiamin und 4-tert.Octylamino-2,6-dichlor-1,3,5-s-triazin, Tris-(2,2,6,6-tetramethyl- 4-piperidyl)-nitrilotriacetat, Tetrakis-(2,2,6,6-tetramethyl-4-piperidyl)-1,2,3,4-butantetraoat, 1,1'-(1,2-Ethandiyl)-bis-(3,3,5,5-tetramethyl-piperazinon).

2.7. Oxalsäurediamide, wie z.B. 4,4'-Di-octyloxy-oxanilid, 2,2'-Di-octyloxy-5,5'-di-tert.-butyl-oxanilid, 2,2'-Di-dodecyloxy-5,5'di-tert.butyl-oxanilid, 2-Ethoxy-2'-ethyl-oxanilid, N,N'-Bis-(3-dimethylaminopropyl)-oxalamid, 2-Ethoxy-5-tert.butyl-2'-ethyloxanilid und dessen Gemisch mit 2-Ethoxy-2'-ethyl-5,4'-di-tert.butyl-oxanilid, Gemische von o- und p-Methoxy- sowie von o- und p-Ethoxy-di-substituierten Oxaniliden.

2.8. 2-(2-Hydroxyphenyl)-1,3,5-triazine, wie z.B. 2,4,6-Tris(2-hydroxy-4-octyloxyphenyl)-1,3,5-triazin, 2-(2-Hydroxy-4-octyloxyphenyl)-4,6-bis-(2,4-dimethylphenyl)-1,3,5-triazin, 2-(2,4-Dihydroxyphenyl)-4,6-bis(2,4-dimethylphenyl)-1,3,5-triazin, 2,4-Bis-(2-hydroxy-4-propyloxyphenyl)-6-(2,4-dimethylphenyl)-1,3,5-triazin, 2-(2-Hydroxy-4-octyloxyphenyl)-4,6-bis(4-methylphenyl)-1,3,5-triazin, 2-(2-Hydroxy-4-dodecyloxyphenyl)-4,6-bis(2,4-dimethylphenyl)-1,3,5-triazin.

3. Metalldesaktivatoren, wie z.B. N,N'-Diphenyloxalsäurediamid, N-Salicylal-N'-salicyloylhydrazin, N,N'-Bis-(salicyloyl)-hydrazin, N,N'-Bis-(3,5-di-tert.butyl-4-hydroxyphenylpropionyl)-hydrazi n, 3-Salicyloylamino-1,2,4-triazol, Bis-(benzyliden)-oxalsäuredihydrazid.

4. Phosphite und Phosphonite, wie z.B. Triphenylphosphit, Diphenylalkylphosphite, Phenyldialkylphosphite, Tris-(nonylphenyl)-phosphit, Trilaurylphosphit, Trioctadecylphosphit, Distearyl-pentaerythritdiphosphit, Tris-(2,4-di-tert.butylphenyl)-phosphit, Diisodecylpentaerythrit-diphosphit, Bis-(2,4-di-tert.butylphenyl)-pentaerythritdiphosphit, Tri-stearyl-sorbit-triphosphit, Tetrakis-(2,4-di-tert.butylphenyl)-4,4'-biphenylen-diphosphonit, 3,9-Bis-(2,4-di-tert.butylphenoxy)-2,4,8,10-tetraoxa-3,9-diphosphaspiro[5.5]undecan.

5. Peroxidzerstörende Verbindungen, wie z.B. Ester der β-Thio-dipropionsäure, beispielsweise der Lauryl-, Stearyl-, Myristyl- oder Tridecylester, Mercaptobenzimidazol, das Zinksalz des 2-Mercaptobenzimidazols, Zink-dibutyl-dithiocarbamat, Dioctadecyldisulfid, Pentaerythrit-tetrakis-(β-dodecylmercapto)-propionat.

6. Polyamidstabilisatoren, wie z.B. Kupfersalze in Kombination mit Jodiden und/oder Phosphorverbindungen und Salze des zweiwertigen Mangans.

7. Basische Co-Stabilisatoren, wie z.B. Melamin, Polyvinylpyrrolidon, Dicyandiamid, Tri-allylcyanurat, Harnstoff-Derivate, Hydrazin-Derivate, Amine, Polyamide, Polyurethane, Alkali- und Erdalkalisalze höherer Fettsäuren, beispielsweise Ca-Stearat, Zn-Stearat, Mg-Stearat, Na-Ricinoleat, K-Palmitat, Antimonbrenzcatechinat oder Zinnbrenzcatechinat.

8. Nukleierungsmittel, wie z.B. 4-tert.Butylbenzoesäure, Adipinsäure, Diphenylessigsäure.

9. Füllstoffe und Verstärkungsmittel, wie z.B. Calciumcarbonat, Silikate, Glasfasern, Asbest, Talk, Kaolin, Glimmer, Bariumsulfat, Metalloxide und -hydroxide, Russ, Graphit.

10. Sonstige Zusätze, wie z.B. Weichmacher, Gleitmittel, Emulgatoren, Pigmente, Optische Aufheller, Flammschutzmittel, Antistatika, Treibmittel.

Handelt es sich bei den erfindungsgemässen Zusammensetzungen um solche auf Basis von Schmierstoffen und Hydraulikflüssigkeiten, können diese ebenfalls weitere Additive enthalten, die zugegeben werden, um gewisse Gebrauchseigenschaften zu verbessern, wie z.B. weitere Antioxidantien, Metalldesaktivatoren, Rostinhibitoren, Viskositäts-Index-Verbesserer, Stockpunkterniedriger, Dispergiermittel/Tenside und Verschleissschutzadditive.

Beispiele für Antioxidantien sind der weiter oben wiedergegebenen Auflistung unter dem Titel "1. Antioxidantien", insbesondere Punkte 1.1 bis 1.10 zu entnehmen. Beispiele für weitere zusätzliche Additive sind die folgenden:

Beispiele für aminische Antioxidantien:

N,N'-Di-isopropyl-p-phenylendiamin, N,N'-Di-sec-butyl-p-phenylendiamin, N,N'-Bis-(1,4-dimethyl-pentyl)-p-phenylendiamin, N,N'-Bis(1-ethyl-3-methyl-pentyl)-p-phenylendiamin, N,N'-Bis(1-methyl-heptyl)-p-phenylendiamin, N,N'-Dicyclohexyl-p-phenylendiamin, N,N'-Diphenyl-p-phenylendiamin, N,N'-Di-(naphthyl-2)-p-phenylendiamin, N-Isopropyl-N'-phenyl-p-phenylendiamin, N-(1,3-Dimethyl-butyl)-N'-phenyl-p-phenylendiamin, N-(1-Methyl-heptyl)-N'-phenyl-p-phenylendiamin, N-Cyclohexyl-N'-phenyl-p-phenylendiamin, 4-(p-Toluol-sulfonamido)-diphenylamin, N,N'-Dimethyl-N,N'-di-sec-butyl-p-phenylendiamin, Diphenylamin, N-Allyldiphenylamin, 4-Isopropoxy-diphenylamin, N-Phenyl-1-naphthylamin, N-Phenyl-2-naphthylamin, octyliertes Diphenylamin, z.B. p,p'-Di-tert-octyldiphenylamin, 4-n-Butylaminophenol, 4-Butyryl-amino-phenol, 4-Nonanoylamino-phenol, 4-Dodecanoylamino-phenol, 4-Octadecanoyl-amino-phenol, Di-(4-methoxyphenyl)-amin, 2,6-Di-tert-butyl-4-dimethylamino-methyl-phenol, 2,4'-Diamino-diphenylmethan, 4,4'-Diamino-diphenylmethan, N,N,N',N'-Tetramethyl-4,4'-diamino-diphenylmethan, 1,2-Di-[(2-methyl-phenyl)-amino]-ethan, 1,2-Di-(phenylamino)-propan, (o-Tolyl)-biguanid, Di-[4-(1',3'-dimethyl-butyl)-phenyl]amin, tert-octyliertes N-Phenyl-1-naphthylamin, Gemisch aus mono- und dialkylierten tert-Butyl/tert-Octyldiphenylaminen, 2,3-Dihydro-3,3-dimethyl-4H-1,4-benzothiazin, Phenothiazin, N-Allylphenothiazin.

Beispiele für weitere Antioxidantien:

Aliphatische oder aromatische Phosphite, Ester der Thiodipropionsäure oder der Thiodiessigsäure, oder Salze der Dithiocarbamid- oder Dithiophosphorsäure.

Beispiele für Metall-Desaktivatoren, z.B. für Kupfer, sind:

Triazole, Benztriazole und deren Derivate, Tolutriazole und deren Derivate, 2-Mercaptobenzthiazol, 2-Mercaptobenztriazol, 2,5-Dimercaptobenztriazol, 2,5-Dimercaptobenzthiadiazol, 5,5'-Methylenbisbenztriazol, 4,5,6,7-Tetrahydrobenztriazol, Salicyliden-propylendiamin, Salicylaminoguanidin und dessen Salze.

Beispiele für Rost-Inhibitoren sind:

a) Organische Säuren, ihre Ester, Metallsalze und Anhydride, z.B.:
N-Oleoyl-sarcosin, Sorbitan-mono-oleat, Blei-naphthenat, Alkenylbernsteinsäureanhydrid, z.B. Dodecenylbernsteinsäure-anhydrid, Alkenylbernsteinsäure-Teilester und -Teilamide, 4-Nonylphenoxy-essigsäure.
b) Stickstoffhaltige Verbindungen, z.B.:
I. Primäre, sekundäre oder tertiäre aliphatische oder cycloaliphatische Amine und Amin-Salze von organischen und anorganischen Säuren, z.B. öllösliche Alkylammoniumcarboxylate.
II. Heterocyclische Verbindungen, z.B.:
Substituierte Imidazoline und Oxazoline.
c) Phosphorhaltige Verbindungen, z.B.:
Aminsalze von Phosphorsäurepartialestern oder Phosphonsäurepartialestern, Zinkdialkyldithiophosphate.
d) Schwefelhaltige Verbindungen, z.B.:
Barium-dinonylnaphthalin-sulfonate, Calciumpetroleum-sulfonate.

Beispiele für Viskositätsindex-Verbesserer sind:

Polyacrylate, Polymethacrylate, Vinylpyrrolidon/Methacrylat-Copolymere, Polyvinyl-pyrrolidone, Polybutene, Olefin-Copolymere, Styrol/Acrylat-Copolymere, Polyether.

Beispiele für Stockpunkterniedriger sind:

Polymethacrylat, alkylierte Naphthalinderivate.

Beispiele für Dispergiermittel/Tenside sind:

Polybutenylbernsteinsäureamide oder -imide, Polybutenylphosphonsäurederivate, basische Magnesium-, Calcium-, und Bariumsulfonate und -phenolate.

Beispiele für Verschleissschutz-Additive sind:

Schwefel und/oder Phosphor und/oder Halogen enthaltende Verbindungen, wie geschwefelte pflanzliche Oele, Zinkdialkyldithiophosphate, Tritolylphosphat, chlorierte Paraffine, Alkyl- und Aryldi- und tri-sulfide, Triphenylphosphorothionate, Diethanolaminomethyltolyltriazol, Di(2-ethylhexyl)aminomethyltolyltriazol.

Die Sulfoxide der vorliegenden Erfindung der Formeln I und II können z.B. durch Oxidation von Verbindungen der Formeln VI bzw. VII erhalten werden. Als Oxidationsmittel kommen z.B. Wasserstoffperoxid oder Percarbonsäuren, organische Hydroperoxide oder andere geeignete organische oder anorganische Oxidationsmittel in Frage, wobei nach in der Literatur einschlägig bekannten Methoden vorgegangen werden kann. Geeignete Percarbonsäuren sind z.B. m-Chlorperbenzoesäure, Peressigsäure oder Trifluorperessigsäure. Als Hydroperoxide lassen sich beispielsweise tert-Butylhydroperoxid oder Cumolperoxid verwenden.

Die Oxidation der Ausgangsprodukte kann auch mit Oxidationsmitteln, die in situ hergestellt werden, erfolgen. Solche Oxidationsmittel sind z.B. Percarbonsäuren, welche im Gemisch aus Wasserstoffperoxid und Carbonsäuren entstehen.

Das Oxidationsmittel wird, je nach gewünschtem Produkt, zweckmässig in stöchiometrischer Menge oder im Überschuss zugegeben. Dabei werden die Reaktionsparameter so gewählt, dass eine möglichst geringe Aufoxidation der Sulfoxide zu den Sulfonen erfolgt. Die Oxidation wird zweckmässig in Gegenwart von Lösungsmitteln ausgeführt. Als Lösungsmittel dienen z.B. nicht oxidierbare organische Lösungsmittel wie z.B. chlorierte Kohlenwasserstoffe, Ketone oder Kohlenwasserstoffe. Geeignete chlorierte Kohlenwasserstoffe sind beispielsweise Methylenchlorid oder Chloroform, geeignete Ketone können z.B. Aceton, Methylethylketon oder Methylisopropylketon sein, als Kohlenwasserstoff eignen sich insbesondere aromatische Kohlenwasserstoffe, beispielsweise Toluol oder Xylol.

Die Reaktionstemperatur kann beispielsweise zwischen -40 und +80°C liegen. Die Reaktionszeiten liegen je nach Temperatur und spezifischer Reaktion beispielsweise zwischen 10 Minuten und 24 Stunden.

Die Ausgangsprodukte, die Verbindungen der Formeln VI und VII, und deren Herstellung sind literaturbekannt und in zahlreichen Patenten, z.B. US-A 4,759,862, US-A 4,857,572, US-A 3,772,390, US-A 4,874,885, US-A 4,820,756 oder FR-A 1569743 beschrieben.

In der Regel fallen bei der Oxidation von Verbindungen der Formeln VI und VII Produktegemische von Verbindungen der Formeln I bzw. II an (an einer oder/und mehreren $-CH_2-S-R_4$-Gruppen oxidierte Produkte; siehe etwa Beispiel 1). Diese Gemische können direkt in den erfindungsgemässen Zusammensetzungen verwendet werden.

Sie können aber auch durch übliche Methoden (wie z.B. Chromatographie, fraktionierte Kristallisation usw.) in die Einzelkomponenten aufgetrennt werden.

Die Herstellung der erfindungsgemässen Mischungen aus Verbindungen der Formeln I bzw. II und solchen der Formeln VI bzw. VII kann z.B. durch Mischen der Einzelverbindungen im gewünschten Mengenverhältnis erfolgen. Bevorzugt werden diese Mischungen aber durch partielle Oxidation von Verbindungen der Formel VI bzw. VII, z.B. nach den oben angegebenen Methoden erhalten, wobei man das Oxidationsmittel in unterstöchiometrischer Menge einsetzt. Seine Menge richtet sich nach dem gewünschten Gehalt an Verbindung der Formel I bzw. II in der Mischung. Sie kann durch einfache Vorversuche bzw. Berechnung leicht ermittelt werden.

Nähere Details können den nachfolgenden Herstellungsbeispielen entnommen werden.

Die nachfolgenden Beispiele erläutern die Erfindung weiter. Angaben in Teilen oder Prozenten beziehen sich, ebenso wie in der übrigen Beschreibung und in den Patentansprüchen, auf das Gewicht, sofern nichts anderes angegeben ist.

Beispiel 1:

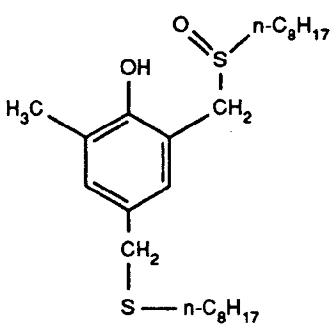

(1a)

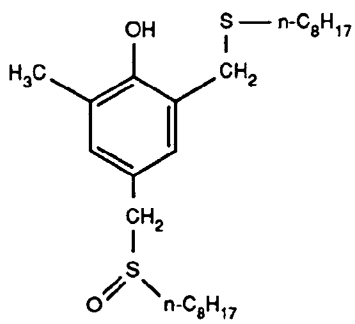

(1b)

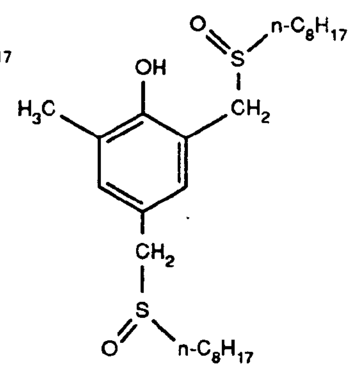

(1c)

127,42 g 2,4-Bis(n-octylthiomethyl)-6-methylphenol werden in 750 ml Aceton gelöst. Zu dieser Lösung werden innerhalb von 10 Minuten bei 0-5°C 17 g 30%iges Wasserstoffperoxid getropft. Nach 2,5-stündigem Rühren bei 0°C wird das Lösungsmittel unter vermindertem Druck abdestilliert. Chromatographie an Kieselgel mit Hexan/Essigester (7:3) als Elutionsmittel ergibt

33,7 g 2-Methyl-6-n-octylsulfinylmethyl-4-n-octylthiomethylphenol (1a) als Oel,

6,2 g 2-Methyl-4-n-octylsulfinylmethyl-6-n-octylthiomethylphenol (1b), Schmelzpunkt 105-106°C, und

16,9 g 2,4-Bis(n-octylsulfinylmethyl)-6-methylphenol (1c), Schmelzpunkt 92°C.

| Elementaranalysenwerte in % | | Berechnet | Gefunden |
|---|---|---|---|
| Beispiel 1a | C | 68,13 | 68,27 |
| | H | 10,06 | 9,92 |
| | S | 14,55 | 14,26 |
| Beispiel 1b | C | 68,13 | 68,28 |
| | H | 10,06 | 10,21 |
| | S | 14,55 | 14,54 |
| Beispiel 1c | C | 65,74 | 65,85 |
| | H | 9,71 | 9,70 |
| | S | 14,04 | 14,01 |

Beispiele 2-18:

Die Verbindungen der Beispiele 2-18 werden nach der folgenden Arbeitsvorschrift hergestellt:
0,1 mol des jeweiligen Bis-alkylthiomethylphenols der Formel VI werden in Aceton oder Methylenchlorid gelöst. In 5-10 Minuten werden bei 0-20°C im Falle der monooxidierten Verbindungen 0,11 mol 30%iges Wasserstoffperoxid zugetropft, für die dioxidierte Verbindung werden 0,22 mol 30%iges Wasserstoffperoxid verwendet. Die Lösung wird während 2 Stunden bei 0°C gerührt. Die Produkte werden durch Säulenchromatographie an Kieselgel in reiner Form erhalten. Die erhaltenen Produkte sind in Tabelle 1 dargestellt.

Tabelle 1:

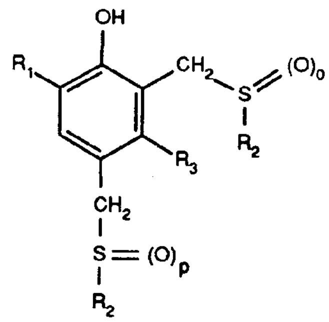

| Bsp. Nr. | $R_1$ | $R_2$ | $R_3$ | o | p | Smp. [°C] | Verhältnis Verbindung der Formel VI/$H_2O_2$ | Elutionsmittel |
|---|---|---|---|---|---|---|---|---|
| 2 | t-Butyl | n-$C_8H_{17}$ | H | 1 | 0 | Öl | 1:1 | D |
| 3 | t-Butyl | n-$C_8H_{17}$ | H | 0 | 1 | Öl | 1:1 | D |
| 4 | t-Butyl | n-$C_8H_{17}$ | H | 1 | 1 | Öl | 1:2 | D |
| 5 | Methyl | n-$C_{12}H_{25}$ | H | 1 | 0 | 47 | 1:1 | A |
| 6 | Methyl | n-$C_{12}H_{25}$ | H | 0 | 1 | 106 | 1:1 | A |
| 7 | Methyl | n-$C_{12}H_{25}$ | H | 1 | 1 | 97 | 1:1 | A |
| 8 | Methyl | $CH_2CH_2OH$ | H | 1 | 0 | Öl | 1:1 | B |
| 9 | Methyl | $CH_2CH_2OH$ | H | 0 | 1 | Öl | 1:1 | B |
| 10 | Methyl | $CH_2CH_2OH$ | H | 1 | 1 | 120 | 1:1 | B |
| 11 | Methyl | $CH_2COO$-2-EH* | H | 1 | 0 | Öl | 1:2 | A |
| 12 | Methyl | $CH_2COO$-2-EH* | H | 0 | 1 | Öl | 1:2 | A |
| 13 | Methyl | $CH_2COO$-2-EH* | H | 1 | 1 | Öl | 1:2 | A |
| 14 | Methyl | n-$C_{12}H_{25}$ | Methyl | 1 | 0 | 74 | 1:1 | A |
| 15 | Methyl | n-$C_{12}H_{25}$ | Methyl | 0 | 1 | 84 | 1:1 | A |
| 16 | t-Butyl | n-$C_{12}H_{25}$ | Methyl | 1 | 1 | 122 | 1:1 | A |
| 17 | t-Butyl | n-$C_{12}H_{25}$ | Methyl | 1 | 0 | 55 | 1:1 | C |
| 18 | t-Butyl | n-$C_{12}H_{25}$ | Methyl | 1 | 1 | 97 | 1:1 | C |

* 2-EH = 2-Ethylhexyl

A = Hexan/Essigester (7:3)
B = Hexan/Aceton (1:2)
C = Hexan/Essigester (9:1)
D = Hexan/Essigester (4:1)

Charakteristische $^1$H-NMR-Signale der Verbindungen der Beispiele 1-18, gemessen in Deuterochloroform bei 100 MHz (Standard: Tetramethylsilan) sind in Tabelle 2 wiedergegeben.

Tabelle 2:

| Bsp. | Aryl-$CH_2$-S=O δ [ppm] | $J_{AB}$ [Hz] | Aryl-$CH_2$SR δ [ppm] | Aryl-$CH_2$S(=O)$CH_2$ δ [ppm] | Aryl-$CH_2$-S-$CH_2$ δ [ppm] | J [Hz] |
|---|---|---|---|---|---|---|
| 1a | 4,38/3,78 | 14 | 3,60 | | 2,25-2,79 (m) | |
| 1b | 3,93/3,76 | 14 | 3,77 | | 2,32-2,66 (m) | |
| 1c | 4,36/3,82 | 14 | | 2,48-2,68 (m) | | |
| | 3,85 (s) | | | | | |
| 2 | 4,33/3,71 | 14 | 3,64 | | 2,35-2,75 (m) | |
| 3 | 3,96/3,79 | 13 | 3,78 | | 2,31-2,65 (m) | |
| 4 | 4,31/3,89 | 14 | | 2,51-2,76 (m) | | |
| | 3,87 (s) | | | | | |
| 5 | 4,39/3,77 | 14 | 3,61 | | 2,25-2,73 (m) | |
| 6 | 3,93/3,77 | 14 | 3,70 | | 2,31-2,63 (m) | |
| 7 | 4,37/3,81 | 14 | | 2,48-2,75 (m) | | |
| | 3,84 (s) | | | | | |
| 8*a | 4,41/3,94 | 13 | 3,62 | 2,82-3,04 (m) | 2,53 (t) | 7 |
| 9*b | 4,73-4,96 (m) | | 3,76 | 2,70-2,95 (m) | 2,52 (t) | 7 |
| 10*c | 4,28/4,08 und | | | 2,60-2,95 (m) | | |
| | 4,27/4,06*d | 12 | | | | |
| | 4,03/3,84 | 13 | | | | |
| 11 | 6,57/4,05 | 14 | 3,72 | 3,68 (s) | 3,07 (s) | |
| 12 | 4,02-4,21 (m) | | 3,86 | 3,64/3,47 J=14 Hz | 3,19 (s) | |
| 13 | 4,57/3,97 und | | | 3,61/3,51 und | | |
| | 4,55/3,95*d | 14 | | 3,59/3,50 J=12 Hz | | |
| | 4,08 (s) | | | 3,68 (s) | | |
| 14 | 4,20 (s) | | 3,66 (s) | | 2,38-2,83 (m) | |
| 15 | 4,09/3,89 | 12 | 3,85 | | 2,43-2,68 (m) | |
| 16 | 4,19 (s) und | | | 2,55-2,86 (m) | | |
| | 3,81-4,05 (m)*d | | | | | |
| 17 | 4,31/4,09 | 14 | 3,69 | | 2,37-2,84 (m) | |
| 18*a | 3,90-4,46 (m)*d | | | 2,56-2,84 (m) | | |

*a bei 360 MHz gemessen

*b bei 360 MHz in einem Gemisch aus Deuterochloroform und 6-fach deuteriertem Dimethylsulfoxid ($d_6$-DMSO) gemessen

*c bei 360 MHz in $d_6$-DMSO gemessen

*d Diastereomerengemisch
s Singulett
m Multiplett
t Triplett

Beispiele 19 und 20:

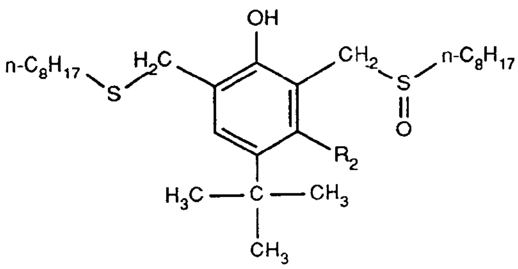

(19)

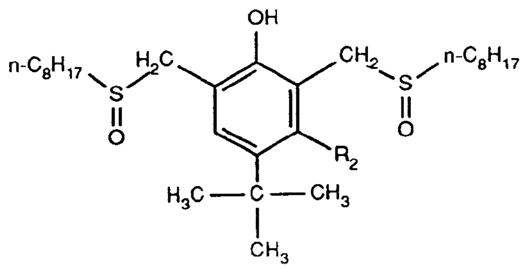

(20)

$R_2$ ist Wasserstoff.

Die Herstellung erfolgt nach der Arbeitsvorschrift der Beispiele 2-18.

Als Elutionsmittel wird ein Gemisch aus Hexan/Essigester im Verhältnis 7:3 verwendet.

| Beispiel | Verhältnis Verbindung der Formel VI/ Wasserstoffperoxid | Ausbeute |
|---|---|---|
| 19 | 1:1 | 38,5 % |
| 20 | 1:2 | 48,8 % |

Beide Verbindungen sind Oele.

Charakteristische ¹H-NMR-Signale der Verbindungen der Beispiele 19 und 20, gemessen in Deuterochloroform bei 100 MHz (Standard: Tetramethylsilan), sind in Tabelle 3 dargestellt.

Tabelle 3:

| Beispiel | Aryl-$C\underline{H}_2S$=O | | Aryl-$C\underline{H}_2SR$ | Aryl-$CH_2S(=O)C\underline{H}_2$ | Aryl-$CH_2$-S-$C\underline{H}_2$ |
|---|---|---|---|---|---|
| | δ [ppm] | $J_{AB}$ [Hz] | δ [ppm] | δ [ppm] | δ [ppm] |
| 19 | 4,34/3,87 | 14 | 3,80 | 2,40-2,74 (m) | |
| 20 | 4,33/3,91 und 4,29/3,88 | 13 | | 2,67 (t) J=7 Hz | |

m = Multiplett

t = Triplett

Beispiel 21:

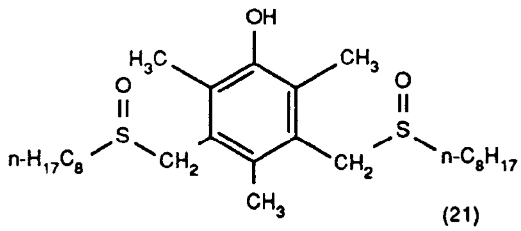

(21)

Zu einer Lösung von 6,0 g 3,5-Bis(n-octylthiomethyl)-2,4,6-trimethylphenol in 130 ml Methylenchlorid werden bei Raumtemperatur 4,81 g m-Chlorperbenzoesäure, in 200 ml Methylenchlorid, getropft. Nach 5-stündigem Erhitzen unter Rückfluss wird bei Raumtemperatur mit gesättigter Natriumhydrogencarbonat-Lösung gewaschen. Der Rückstand aus den vereinigten organischen Phasen wird über Kieselgel filtriert (Elutionsmittel: Methylenchlorid/Methanol 40:1). Nach Umkristallisation aus Methylenchlorid/Hexan werden 5,1 g 3,5-Bis(n-octylsulfinylmethyl)-2,4,6-trimethylphenol erhalten. Der Schmelzpunkt liegt bei 175°C.

Elementaranalysenwerte:

| | | | |
|---|---|---|---|
| Berechnet: | C 66,89 % | Gefunden: | C 66,86 % |
| | H 9,98 % | | H 10,10 % |
| | S 13,23 % | | S 13,24 % |

Beispiele 22-24:

In Analogie zu der im Beispiel 21 angegebenen Arbeitsweise werden die in Tabelle 4 wiedergegebenen Verbindungen hergestellt.

Tabelle 4:

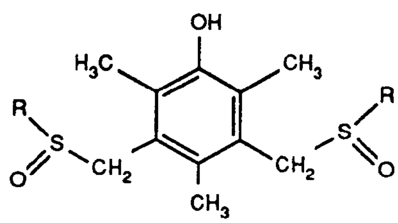

| Beispiel | R | Schmelzpunkt [°C] | Ausbeute [%] |
|---|---|---|---|
| 22 | t-$C_8H_{17}$*1 | 137 | 82,0 |
| 23 | n-$C_{12}H_{25}$ | 165 | 92,0 |
| 24 | $CH_2COO$-2-EH*2 | 92 | 88,0 |

*1 t-$C_8H_{17}$ = 1,1,3,3-Tetramethylbutyl

*2 2-EH = 2-Ethylhexyl

Charakteristische $^1H$-NMR-Signale der Verbindungen der Beispiele 21-24, gemessen in Deuterochloroform bei 100 MHz (Standard: Tetramethylsilan), sind in Tabelle 5 wiedergegeben.

Tabelle 5:

| Beispiel | Aryl-C$H_2$-S=O [ppm] | $J_{AB}$ [Hz] | Aryl-$CH_2$-S(=O)C$H_2$ |
|---|---|---|---|
| 21 | 4,28/4,00 | 13 | 2,54-2,95 (m) |
| 22 | 4,08/3,70 und 4,05/3,78* | 13<br>12,5 | - |
| 23 | 4,30/4,04 | 13 | 2,50-2,95 (m) |
| 24 | 4,48/4,28 | 12,5 | 3,80 (s) |

* Diastereomerengemisch
m = Multiplett
s = Singulett

Beispiel 25: Stabilisierung von Polybutadien-Kautschuk (Brabender Test)

100 Teile Polybutadien, welches mit 0,36 % 2,6-Di-tert-butyl-p-cresol vorstabilisiert ist, wird zusätzlich mit 0,15 % des zu prüfenden Stabilisators in einem Brabender Plastographen bei 160°C und 60 Umdrehungen pro Minute während 30 Minuten geknetet.

Das Gerät arbeitet nach folgendem Prinzip:
Zwei Knetwalzen, die in einer thermostatisch beheizten Knetkammer rotieren, werden von einem pendelnd gelagerten Motor angetrieben. Zur Ueberwindung des Fliesswiderstandes des Kunststoffes, der sich in der Knetkammer befindet, ist ein bestimmtes Drehmoment erforderlich, das mit dem Drehmomentspendel zur Anzeige gebracht wird und zeitabhängig mitgeschrieben wird. Aus dem Verlauf der Drehmomentskurve wird die Induktionszeit ermittelt, d.h. die Knetzeit in Minuten bis zum Anstieg des Drehmoments um 100 mp nach dem Drehmomentminimum. Die Farbintensität wird nach dem "Yellowness Index" (YI) nach ASTM D 1925 quantifiziert. Höhere Zahlen bedeuten intensiveren Gelbeindruck. Die Versuche zeigen, dass durch den zugesetzten Stabilisator die Farbentwicklung wirksam unterdrückt und der Fliesswiderstand wesentlich länger konstant gehalten wird.

Die Ergebnisse sind in Tabelle 6 zusammengefasst.

Tabelle 6:

| Stabilisator aus Beispiel | Induktionszeit [min] | Yellowness Index |
|---|---|---|
| 1a | 30 | 11 |
| 1b | 30 | 12 |
| 2 | 30 | 13 |
| 5 | 30 | 17 |
| 14 | 30 | 10 |
| 19 | 27 | 13 |
| Kontrolle* | 9,5 | 31 |

* die Kontrolle enthält keinen erfindungsgemässen Stabilisator

Beispiele 26-35:

Die in der Tabelle 7 aufgeführten erfindungsgemässen Stabilisatorgemische werden durch Mischen der Verbindungen gemäss Beispiel 1a, 1b und/oder 1c mit 2,4-Bis(n-octylthiomethyl)-6-methylphenol in den angegebenen Mengenverhältnissen hergestellt.

Tabelle 7:

| Beispiel | Zusammensetzung in Gew.-% 1a | 1b | 1c | 2,4-Bis(n-octylthiomethyl)-6-methylphenol |
|---|---|---|---|---|
| 26 | 1 | - | - | 99 |
| 27 | - | 1 | - | 99 |
| 28 | - | - | 1 | 99 |
| 29 | 10 | - | - | 90 |
| 30 | 50 | - | - | 50 |
| 31 | - | 50 | - | 50 |
| 32 | - | 90 | - | 10 |
| 33 | 1 | - | 1 | 98 |
| 34 | 10 | 10 | - | 80 |
| 35 | 4 | 1 | 5 | 90 |

Beispiel 36:

Man wiederholt Beispiel 25, setzt jedoch an Stelle von 0,15 % der dort geprüften Stabilisatoren die gleiche Menge der Mischungen gemäss Beispiel 26-35 ein.

Diese Mischungen verlängern die Induktionszeit ebenfalls beträchtlich und der Yellowness-Index des Polymeren wird deutlich verbessert.

## Patentansprüche

1. Verbindungen der Formeln I und II

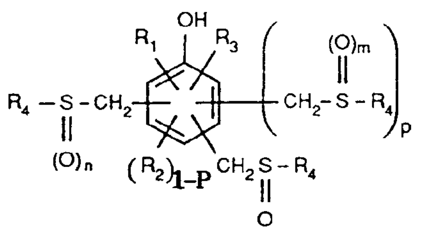

(I)

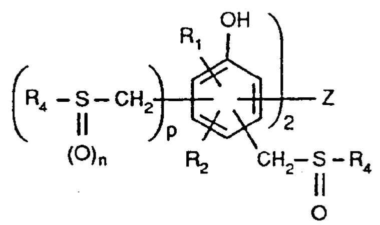

(II)

worin
$R_1$ Wasserstoff, $C_1$-$C_{18}$-Alkyl, Cyclohexyl oder $C_7$-$C_9$-Phenylalkyl bedeutet,
$R_2$ und $R_3$ unabhängig voneinander für Wasserstoff oder Methyl stehen,
$R_4$ für $C_4$-$C_{18}$-Alkyl, Phenyl, $C_7$-$C_9$-Phenylalkyl, mit $C_1$-$C_4$-Alkyl substituiertes Phenyl, Hydroxyethyl oder einen Rest $-(CH_2)_rCOOR_5$ steht, worin r 1 oder 2 entspricht und $R_5$ $C_1$-$C_{18}$-Alkyl bedeutet,
Z für Schwefel,

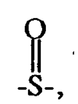

$>CR_2R_3$, wobei $R_2$ und $R_3$ die oben angegebene Bedeutung haben, oder für eine direkte Bindung steht und n, m und p unabhängig voneinander die Werte 0 oder 1 darstellen.

2. Verbindungen gemäss Anspruch 1, worin p 0 ist und $R_4$ für $C_4$-$C_{18}$-Alkyl, Phenyl, $C_7$-$C_9$-Phenylalkyl, Hydroxyethyl oder einen Rest $-CH_2COOR_5$ steht, wobei $R_5$ $C_1$-$C_{18}$-Alkyl ist.

3. Verbindungen der Formel I gemäss Anspruch 1.

4. Verbindungen gemäss Anspruch 3, worin p 0 ist, $R_1$ für Wasserstoff, $C_1$-$C_4$-Alkyl oder Cyclohexyl steht und $R_4$ $C_4$-$C_{18}$-Alkyl, insbesondere $C_8$-$C_{12}$-Alkyl, Benzyl, Phenyl, Hydroxyethyl oder $-CH_2COOR_5$ bedeutet und $R_5$ $C_1$-$C_{18}$-Alkyl entspricht.

5. Verbindungen gemäss Anspruch 3, worin p für 0 steht und die Gruppen

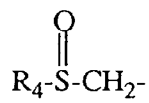

und

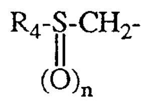

in 2,4-, 2,6- oder 3,5-Stellung zur OH-Gruppe stehen.

**6.** Verbindungen gemäss Anspruch 5, worin $R_1$ Wasserstoff oder $C_1$-$C_4$-Alkyl ist und $R_4$ für ein $C_8$-$C_{12}$-Alkyl, Hydroxyethyl oder einen Rest -$CH_2COOR_5$ steht, wobei $R_5$ $C_8$-$C_{12}$-Alkyl bedeutet.

**7.** Verbindungen gemäss Anspruch 1 der Formel III

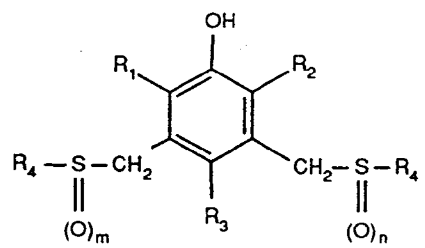

(III),

worin $R_1$ Wasserstoff, $C_1$-$C_4$-Alkyl oder Cyclohexyl ist und $R_2$, $R_3$ und $R_4$ wie in Anspruch 2 definiert sind und n und m unabhängig voneinander 0 oder 1 darstellen, wobei $n+m \geqq 1$ ist.

**8.** Verbindungen gemäss Anspruch 1 der Formel IV

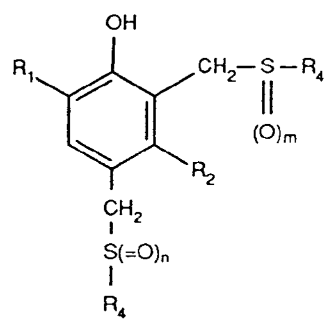

(IV),

worin $R_1$ $C_1$-$C_4$-Alkyl bedeutet, $R_2$ und $R_4$ wie in Anspruch 2 definiert sind und n und m unabhängig voneinander 0 oder 1 darstellen, wobei $n+m \geqq 1$ ist.

**9.** Verbindungen gemäss Anspruch 1 der Formel V

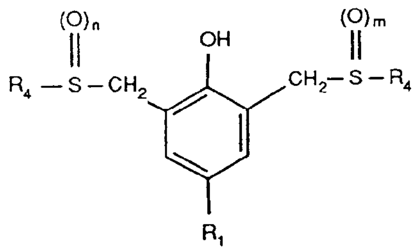 (V),

worin $R_1$ $C_1$-$C_{12}$-Alkyl ist, $R_4$ wie in Anspruch 2 definiert ist und n und m unabhängig voneinander 0 oder 1 darstellen, wobei $n+m \geqq 1$ ist.

**10.** Mischungen von mindestens einer der in den Ansprüchen 1 bis 9 definierten Verbindungen mit mindestens einer Verbindung der Formel VI oder/und VII

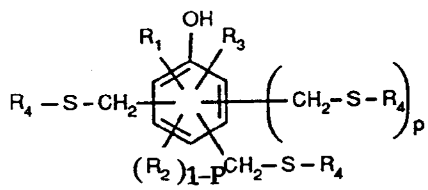 (VI)

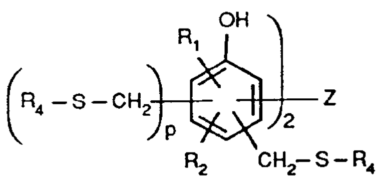 (VII),

worin $R_1$, $R_2$, $R_3$, $R_4$, Z und p die in Anspruch 1 gegebene Bedeutung haben.

**11.** Mischungen nach Anspruch 10, worin sich die Komponenten der Formeln I bzw. II von jenen der Formeln VI bzw. VII nur dadurch unterscheiden, dass letztere an Stelle der

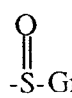Gruppierungen

-S-Gruppen enthalten, sonst aber die gleiche Struktur besitzen wie erstere.

**12.** Mischungen nach Anspruch 10, erhältlich durch partielle Oxidation mindestens einer Verbindung der Formeln VI und/oder VII.

**13.** Mischungen nach Anspruch 10 oder 11, worin das Verhältnis der Verbindungen der Formel I bzw. II zu jenen der Formel VI bzw. VII 0,5:99,5 bis 99,5:0,5 beträgt.

**14.** Zusammensetzungen enthaltend ein gegen oxidativen, thermischen und/oder lichtinduzierten Abbau empfindliches organisches Material und mindestens eine Verbindung der Formel I und/oder II nach Anspruch 1 oder mindestens eine in Anspruch 10 definierte Mischung.

**15.** Zusammensetzung nach Anspruch 14 enthaltend mindestens eine der in den Ansprüchen 2 bis 9 definierte Verbindung.

**16.** Zusammensetzung nach Anspruch 14, worin das organische Material ein organisches, vorzugsweise synthetisches, Polymer, ein Schmierstoff oder eine Hydraulikflüssigkeit ist.

17. Zusammensetzung nach Anspruch 16, worin das organische Material ein Elastomer oder ein Schmierstoff ist.

18. Zusammensetzung nach Anspruch 14 enthaltend mindestens eine Verbindung der Formel I oder Gemische von Verbindungen der Formeln I und VI.

19. Verwendung von Verbindungen der Formel I oder II nach Anspruch 1 oder von Mischungen nach Anspruch 10 als Stabilisatoren in einem gegen oxidativen, thermischen und/oder lichtinduzierten Abbau empfindlichen organischen Material.

20. Verwendung nach Anspruch 19, worin das organische Material ein organisches Polymer, ein Schmierstoff oder eine Hydraulikflüssigkeit ist.

21. Verwendung von Verbindungen nach Anspruch 19, worin das organische Material ein Elastomer oder ein Schmierstoff ist.

## Claims

1. A compound of formula I or II

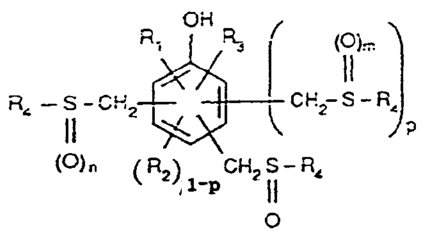

(I)

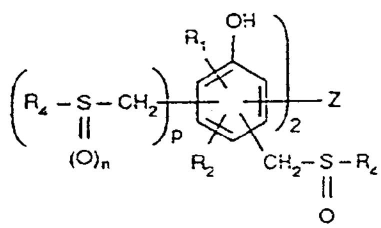

(II)

in which
$R_1$ is hydrogen, $C_1$-$C_{18}$alkyl, cyclohexyl or $C_7$-$C_9$phenylalkyl,
$R_2$ and $R_3$ are each independently of the other hydrogen or methyl,
$R_4$ is $C_4$-$C_{18}$alkyl, phenyl, $C_7$-$C_9$phenylalkyl, $C_1$-$C_4$alkyl-substituted phenyl, hydroxyethyl or a radical $-(CH_2)_rCOOR_5$, in which r is 1 or 2 and $R_5$ is $C_1$-$C_{18}$alkyl,
Z is sulfur,

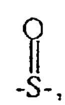

$>CR_2R_3$, where $R_2$ and $R_3$ are as defined above, or are a direct bond, and n, m and p are each independently of the other 0 or 1.

2. A compound according to claim 1, in which p is 0, $R_4$ is $C_4$-$C_{18}$alkyl, phenyl, $C_7$-$C_9$phenylalkyl, hydroxyethyl or a radical $-CH_2COOR_5$, where $R_5$ is $C_1$-$C_{18}$alkyl.

3. A compound of formula I according to claim 1.

4. A compound according to claim 3, in which p is 0, $R_1$ is hydrogen, $C_1$-$C_4$alkyl or cyclohexyl, and $R_4$ is $C_4$-$C_{18}$alkyl, especially $C_8$-$C_{12}$alkyl, benzyl, phenyl, hydroxyethyl or $-CH_2COOR_5$, and $R_5$ is $C_1$-$C_{18}$alkyl.

5. A compound according to claim 3, in which p is 0 and the groups

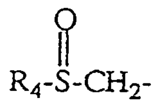
and
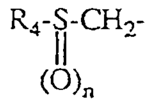

are in 2,4-, 2,6- or 3,5-position to the OH group.

6. A compound according to claim 5, in which $R_1$ is hydrogen or $C_1$-$C_4$alkyl and $R_4$ is $C_8$-$C_{12}$alkyl, hydroxyethyl or a radical $-CH_2COOR_5$, where $R_5$ is $C_8$-$C_{12}$alkyl.

7. A compound according to claim 1 of formula III

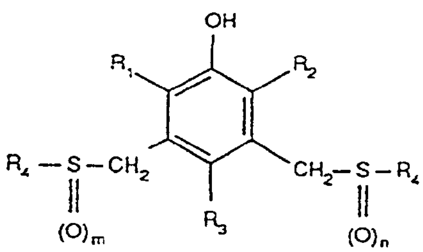
(III),

in which $R_1$ is hydrogen, $C_1$-$C_4$alkyl or cyclohexyl, and $R_2$, $R_3$ and $R_4$ are as defined in claim 2, and n and m are each independently of the other 0 or 1, with the proviso that n + m is ≧ 1.

8. A compound according to claim 1 of formula IV

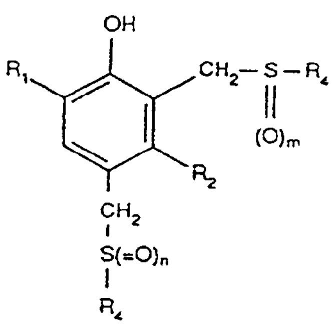
(IV),

in which $R_1$ is $C_1$-$C_4$alkyl, $R_2$ and $R_4$ are as defined in claim 2, and n and m are each independently of the other 0 or 1, with the proviso that n + m is ≧ 1.

9. A compound according to claim 1 of formula V

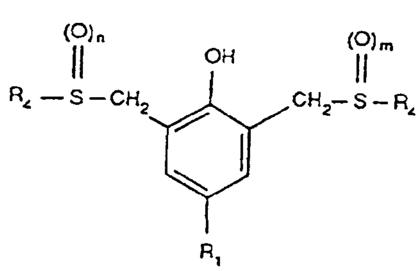
(V),

in which $R_1$ is $C_1$-$C_{12}$alkyl, $R_4$ is as defined in claim 2, and n and m are each independently of the other 0 or 1, with the proviso that n + m is ≧ 1.

**10.** A mixture of at least one compound as defined in any one of claims 1 to 9 with at least one compound of formula VI and/or VII

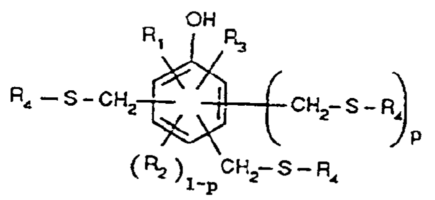

(VI)

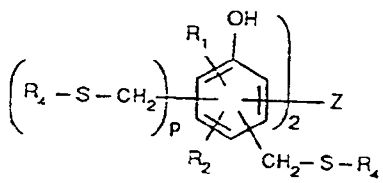

(VII),

in which $R_1$, $R_2$, $R_3$, $R_4$, Z and p are as defined in claim 1.

**11.** A mixture according to claim 10, wherein the components of formulae I and II differ from those of formulae VI and VII respectively only in the fact that the latter contain -S- groups in place of the

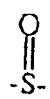

groupings, but otherwise have the same structure as the former.

**12.** A mixture according to claim 10, which is obtainable by partial oxidation of at least one compound of formula VI and/or VII.

**13.** A mixture according to claim 10 or claim 11, wherein the ratio of the compound of formula I or II to the compound of formula VI or VII respectively is 0.5:99.5 to 99.5:0.5.

**14.** A composition comprising an organic material which is sensitive to oxidative, thermal and/or light-induced degradation and at least one compound of formula I and/or II according to claim 1 or at least one mixture as defined in claim 10.

**15.** A composition according to claim 14, comprising at least one compound as defined in any one of claims 2 to 9.

**16.** A composition according to claim 14, wherein the organic material is an organic, preferably synthetic, polymer, a lubricant composition or a hydraulic fluid.

**17.** A composition according to claim 16, wherein the organic material is an elastomer or a lubricant composition.

**18.** A composition according to claim 14, comprising at least one compound of formula I or a mixture of compounds of formulae I and VI.

**19.** Use of a compound of formula I or II according to claim 1 or of a mixture according to claim 10 as stabilizer in an organic material which is sensitive to oxidative, thermal and/or light-induced degradation.

**20.** Use according to claim 19, wherein the organic material is an organic polymer, a lubricant composition or

a hydraulic fluid.

21. Use of a compound according to claim 19, wherein the organic material is an elastomer or a lubricant composition.

**Revendications**

1. Composés de formule I et II

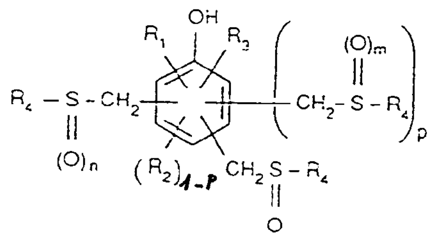

(I)

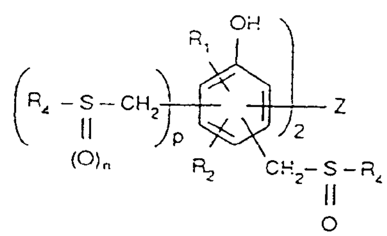

(II)

dans lesquelles
$R_1$ signifie hydrogène, alkyle en $C_1$-$C_{18}$, cyclohexyle ou phénylalkyle en $C_7$-$C_9$,
$R_2$ et $R_3$ sont indépendamment l'un de l'autre hydrogène ou méthyle,
$R_4$ signifie alkyle en $C_4$-$C_{18}$, phényle, phénylalkyle en $C_7$-$C_9$, phényle substitué par alkyle en $C_1$-$C_4$, hydroxyéthyle ou un reste $-(CH_2)_rCOOR_5$, où r est 1 ou 2 et $R_5$ est alkyle en $C_1$-$C_{18}$,
Z est le soufre,

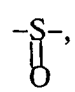

$>CR_2R_3$, où $R_2$ et $R_3$ ont la signification donnée ci-dessus, ou une liaison directe, et n, m, et p représentent indépendamment l'un de l'autre les valeurs 0 ou 1.

2. Composés selon la revendication 1, dans lesquels p est 0 et $R_4$ est alkyle en $C_4$-$C_{18}$, phényle, phénylalkyle en $C_7$-$C_9$, hydroxyéthyle ou un reste $-CH_2COOR_5$, où $R_5$ est alkyle en $C_1$-$C_{18}$.

3. Composés de formule I selon la revendication 1.

4. Composés selon la revendication 3, dans lesquels p est 0, $R_1$ est hydrogène, alkyle en $C_1$-$C_4$ ou cyclohexyle, $R_4$ est alkyle en $C_4$-$C_{18}$, en particulier alkyle en $C_8$-$C_{12}$, benzyle, phényle, hydroxyéthyle ou $-CH_2COOR_5$, et $R_5$ correspond à alkyle en $C_1$-$C_{18}$.

5. Composés selon la revendication 3, dans lesquels p est 0 et les groupes

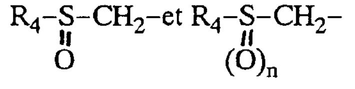

sont situés en position 2,4, 2,6 ou 3,5 par rapport au groupe OH.

**6.** Composés selon la revendication 5, dans lesquels $R_1$ est hydrogène ou alkyle en $C_1$-$C_4$, et $R_4$ est alkyle en $C_8$-$C_{12}$, hydroxyéthyle ou un reste $-CH_2COOR_5$ où $R_5$ est alkyle en $C_8$-$C_{12}$.

**7.** Composés selon la revendication 1, de formule III

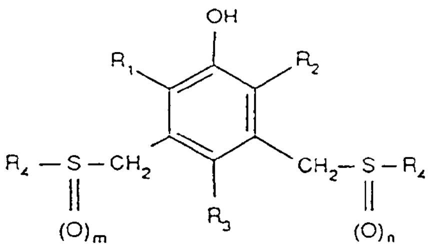

(III),

dans laquelle $R_1$ est hydrogène, alkyle en $C_1$-$C_4$ ou cyclohexyle et $R_2$, $R_3$ et $R_4$ sont tels que définis dans la revendication 2, et n et m signifient indépendamment l'un de l'autre 0 ou 1, avec $n + m \geqq 1$.

**8.** Composés selon la revendication 1, de formule IV

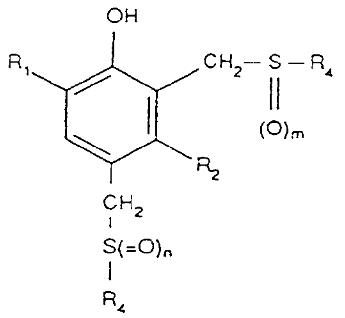

(IV),

dans laquelle $R_1$ est alkyle en $C_1$-$C_4$, $R_2$ et $R_4$ sont tels que définis dans la revendication 2 et n et m signifient indépendamment l'un de l'autre 0 ou 1, avec $n + m \geqq 1$.

**9.** Composés selon la revendication 1, de formule V

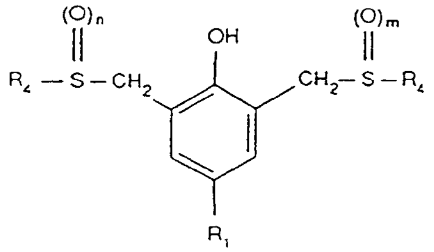

(V),

dans laquelle $R_1$ est alkyle en $C_1$-$C_{12}$, $R_4$ est tel que défini dans la revendication 2 et n et m signifient indépendamment l'un de l'autre 0 ou 1, avec $n + m \geqq 1$.

**10.** Mélanges d'au moins un des composés définis dans l'une des revendications 1 à 9 avec au moins un composé de formule VI et/ou VII

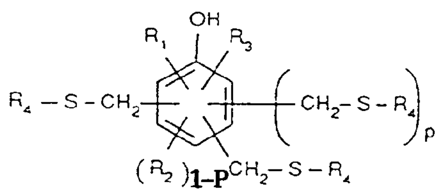

(VI)

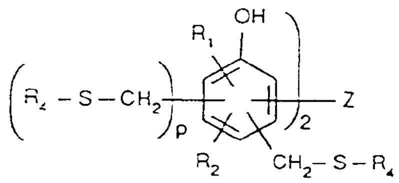

(VII),

dans lesquelles $R_1$, $R_2$, $R_3$, $R_4$, Z et p ont la signification indiquée dans la revendication 1.

**11.** Mélanges selon la revendication 10, dans lesquels les constituants de formule I et II ne diffèrent respectivement de ceux de formule VI et VII que par le fait que ces derniers contiennent des groupes -S- à la place des groupements

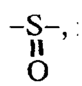

mais ont par ailleurs la même structure que les premiers.

**12.** Mélanges selon la revendication 10, pouvant être obtenus par oxydation partielle d'au moins un composé de formule VI et/ou VII.

**13.** Mélanges selon la revendication 10 ou 11, dans lesquels le rapport des composés de formule I et II à ceux de formule VI et VII est compris entre 0,5:99,5 et 99,5:0,5.

**14.** Compositions contenant une matière organique sensible à la dégradation due à l'oxydation, la chaleur et/ou la lumière et au moins un composé de formule I et/ou II selon la revendication 1 ou au moins un mélange défini dans la revendication 10.

**15.** Composition selon la revendication 14, contenant au moins un composé défini dans l'une des revendications 2 à 9.

**16.** Composition selon la revendication 14, dans laquelle la matière organique est un polymère organique, de préférence synthétique, un lubrifiant ou un fluide hydraulique.

**17.** Composition selon la revendication 16, dans laquelle la matière organique est un élastomère ou un lubrifiant.

**18.** Composition selon la revendication 14, contenant au moins un composé de formule I ou des mélanges de composés de formule I et VI.

**19.** Utilisation de composés de formule I ou II selon la revendication 1 ou de mélanges selon la revendication 10 comme stabilisants dans une matière organique sensible à la dégradation due à l'oxydation, la chaleur et/ou la lumière.

**20.** Utilisation selon la revendication 19, dans laquelle la matière organique est un polymère organique, un lubrifiant ou un fluide hydraulique.

**21.** Utilisation de composés selon la revendication 19, dans laquelle la matière organique est un élastomère ou un lubrifiant.